# EUROPEAN PATENT APPLICATION

(11) **EP 1 498 478 A1**
(43) Date of publication of application: **19.01.2005**
(21) Application number: 03725590.8
(22) Date of filing: 16.04.2003
(51) Int. Cl.: C12N 5/02, C12N 5/06, C12N 15/09, C12Q 1/02, A61K 35/39, A61P 3/10

(54) **METHOD OF FORMING PANCREATIC BETA CELLS FROM MESENCHYMAL CELLS**

(30) Priority: 17.04.2002 JP 2002115201
(71) Applicant: OTSUKA PHARMACEUTICAL CO., LTD., Chiyoda-ku Tokyo 101-8535 (JP)
(72) Inventor: UMEZAWA, Akihiro, Matsudo-shi, Chiba 270-0014 (JP); IZAWA, Yoshikane, Nishitokyo-shi, Tokyo 202-0002 (JP)
(74) Representative: HOFFMANN EITLE
(86) International application number: PCT/JP2003/004812
(87) International publication number: WO 2003/087349

(57) **Abstract**

It is intended to provide a method of forming pancreatic β cells from mesenchymal cells characterized by comprising using mammal-origin mesenchymal cells as starting cells, culturing these cells in the presence of, for example, a pancreatic β cell-forming agent, and selecting and separating the thus obtained pancreatic βcells with the use of a gene expressed specifically in such cells as a selection marker; a remedy for glucose intolerance which comprises pancreatic β cells obtained by the above method as the active ingredient; a pancreatic β cell-forming agent such as a cytokine to be used in the above method; a method of screening a candidate compound promoting the formation of pancreatic β cells from mesenchymal cells; and a pancreatic β cell formation promoter obtained by this screening method.

SEQUENCE LISTING

<110> Otsuka Pharmaceutical Co., Ltd.

<120> Pdx-1/IPF-1

<130> P03-19

<160> 1

<170> PatentIn version 3.1

<210> 1
<211> 939
<212> DNA
<213> M.musculus

<400> 1
accatgaaca gtgaggagca gtactacgcg gccacacagc tctacaagga cccgtgcgca 60

ttccagaggg gcccggtgcc agagttcagc gctaaccccc ctgcgtgcct gtacatgggc 120

cgccagcccc cacctccgcc gccaccccag tttacaagct cgctgggatc actggagcag 180

ggaagtcctc cggacatctc cccatacgaa gtgcccccgc tcgcctccga cgacccggct 240

ggcgctcacc tccaccacca ccttccagct cagctcgggc tcgcccatcc acctcccgga 300

cctttcccga atggaaccga gcctgggggc ctggaagagc ccaaccgcgt ccagctccct 360

ttcccgtgga tgaaatccac caaagctcac gcgtggaaag gccagtgggc aggaggtgct 420

tacacagcgg aacccgagga aaacaagagg acccgtactg cctacacccg ggcgcagctg 480

ctggagctgg agaaggaatt cttatttaac aaatacatct cccggccccg ccgggtggag 540

ctggcagtga tgttgaactt gaccgagaga cacatcaaaa tctggttcca aaaccgtcgc 600

atgaagtgga aaaaagagga agataagaaa cgtagtagcg ggaccccgag tgggggcggt 660

gggggcgaag agccggagca agattgtgcg gtgacctcgg gcgaggagct gctggcagtg 720

ccaccgctgc cacctcccgg aggtgccgtg cccccaggcg tcccagctgc agtccgggag 780

ggcctactgc cttcgggcct tagcgtgtcg ccacagccct ccagcatcgc gccactgcga 840

ccgcaggaac cccggtgagg acagcagtct gagggtgagc gggtctggga cccagagtgt 900

ggacgtggga gcgggcagct ggataaggga acttaacct 939

## Description

### TECHNICAL FIELD

The present invention relates to a method of forming pancreatic β cells from mesenchymal cells. The invention also relates to a pancreatic β cell-forming agent capable of causing the formation of pancreatic β cells from mesenchymal cells. Further, the invention relates to a method of screening candidate substances capable of promoting the formation of pancreatic β cells from mesenchymal cells. In addition, the invention relates to a method of treatment of and a therapeutic agent for impaired glucose tolerance which utilize the pancreatic β cells obtained by the method of forming pancreatic β cells from mesenchymal cells.

### BACKGROUND ART

In the fetal stage, pancreatic β cells are actively dividing while producing insulin but, when inflammation occurs or as the host becomes older after birth (aging), for instance, they are gradually degenerated and necrotized. Therefore, the number of pancreatic β cells remaining in the body of a patient in such a condition is small and this causes decreases in blood insulin level or impaired glucose tolerance.

In the art, diseases due to such impaired glucose tolerance accompanying degeneration and necrosis of pancreatic β cells as caused by inflammation, for instance, are treated mainly by such symptomatic therapy as administration of insulin in increased doses. On the contrary, transplantation of pancreatic β cells appears to serve as a radical treatment against severe impaired glucose tolerance-due diseases but is not yet in general use in medical treatment because of such problems as shortage of organ donors, difficulty in brain death judgment, rejection reaction, and increased fees for medical treatment. In fact, impaired glucose tolerance-due diseases, such as diabetes, are important risk factors for nephropathy, neuropathy, retinopathy, ischemic heart diseases, and hypertension, among others. If pancreatic β cells once lost can be regenerated, this is considered to be conducive to a great advance in medicine and welfare.

In the art, insulinoma cells and RIN cells are known as cell lines retaining the properties of pancreatic β cells [Clark, S.A. et al., Diabetes, 46 (10), 1663 (1997)]. However, transplantation of these cells causes tumor formation and therefore these cells have a problem in that they are not suited for use in cell transplantation. Under such circumstances, the following three methods have been devised for reconstructing pancreatic β cells.

The first method comprises converting cells other than pancreatic β cells into pancreatic β cells. This method was conceived by analogy with the fact that Pdx-1/IPF-1, for instance, is introduced into hepatocytes, these cells are converted to β cell-like cells [Nature Med., 6(5): 568, 2001].

The second method comprises providing pancreatic β cells again with the ability to divide. This is based on the phenomenon of the division of β cells in the fetal period and on the phenomenon of the division of β cells in excised pancreas.

The third method comprises inducing pancreatic β cells from undifferentiated stem cells. It is already known that pancreatic p cells can be derived from embryonic stem cells (ES cells) [Science, 292: 1389-1393, 2001; Diabetes, 49: 157-162, 2000; Diabetes, 50: 1691-1697, 2001]. However, these ES cells have a drawback in that when transplanted into a living body, they form carcinoma and, further, there are antigenicity and other problems. A method of deriving pancreatic β cells from stem cells occurring in Langerhans' islands in the pancreas of adults is also known [Diabetes, 50: 521-533, 2001].

For applying such ES cells to an actual medical treatment, at least a technology of highly purifying precursors of pancreatic β cells or pancreatic β cells is indispensable. As for the antigenicity problem, the possibility of solving it by the technology of cloning is suggested but the technology requires a complicated and troublesome procedure, hence it is not easy to apply it to general medical treatment.

A method comprising obtaining precursors of pancreatic β cells, which are undifferentiated cells, and using them for transplantation has also been conceived and it is reported that the precursor cells effectively function as pancreatic β cells in an experiment using animals [Ramiya, V. K., et al., Nat. Med., 6(3), 278 (2000); Soria, B., et al., Diabetes, 49(2), 157 (2000)]. The report suggests that a cell therapy technique comprising collecting the bone marrow fluid from the patient him/herself and, after in vitro cell culture and medicinal treatment, transplanting the same into a site where pancreatic β cells are impaired may possibly become a realistic means of medical treatment.

In the adult bone marrow, there are not only hematopoietic stem cells and vascular stem cells but also mesenchymal stem cells, and it is reported that those mesenchymal stem cells can be induced to differentiate into osteocytes, chondrocytes, tendon cells, ligament cells, skeletal muscle cells, adipocytes, interstitial cells, and liver oval cells [Science, 284, 143-147 (1999); Science, 284, 1168-1170 (1999)].

Among the methods of collecting desired cells from a tissue of a living organism, there is known a method which uses antibodies capable of recognizing various surface antigens specific to the desired cells. As for the surface antigens specific to the above cells, the following findings have been obtained for the time being. Thus, for instance, immature hematopoietic stem cells have the characteristics CD34+/CD38-/HLA-DR-/CD90(Thy-1)+ and, with the differentiation of the hematopoietic stem cells, CD38 is expressed and CD90(Thy-1) disappears [Tanpakushitu, Kakusan, Koso (Protein, Nucleic Acid, Enzyme), Vol. 45, No. 13, 2056-2062 (2000)]. Vascular endothelial cells are expressing such markers as CD34, CD31, Flk-1, Tie-2 and E-selectin [Bunshi Shin-Kekkan Byo (Molecular Cardiovascular Diseases), Vol. 1, No. 3, 294-302 (2000)], and bone marrow mesenchymal stem cells are expressing such markers as CD90, CD105 and CD140 [Science, 284, 143-147 (1999); Science, 284, 1168-1170 (1999)]. As regards mesenchymal stem cells capable of inducing pancreatic β cells, however, no such specific surface markers as mentioned above have been made clear as yet.

### DISCLOSURE OF INVENTION

As discussed above, a technology enabling safer and more reliable treatment of impaired glucose tolerance-due diseases, such as diabetes, in particular a technology of establishing pancreatic β cells capable of being safely transplanted and/or regenerating pancreatic β cells, and a technology accompanying thereto of controlling the proliferation/differentiation of pancreatic β cells as well as elucidation and identification of cytokines or transcription factors to serve in such controlling have been demanded in the relevant field of the art.

In the course of intensive investigations made by them in an attempt to provide a novel technology effective in the treatment of impaired glucose tolerance-due diseases which has been demanded in the relevant field of the art, as mentioned above, the present inventors obtained the following findings.

Thus, first, mouse bone marrow-derived mesenchymal cells were separated from one another to a single cell level, and a plurality of cell lines capable of forming pancreatic β cells were obtained. Then, the cell lines obtained were labeled with a retrovirus vector expressing the GFP (green fluorescent protein) and, by following each single cell under a fluorescence microscope, it was found that the above-mentioned mesenchymal cells are pluripotent stem cells capable of being induced to differentiate into at least two kinds of cells, namely pancreatic β cells and nerve cells. Further, it was found that said stem cells stochastically differentiate into two series, namely pancreatic β cells and nerve cells and, in addition, those transcription factors, humoral factors and matrices which are involved in this differentiation as differentiation inducers (pancreatic β cell forming agents) could be identified. Then, by transplantation experiments, it was established that the above mesenchymal cells turn into the bone, cartilage, fat, heart muscle, skeletal muscle, nerve, and blood vessel. In view of these results, the present inventors found that, unlike those stem cells hematopoietic which occur in the bone marrow and are known to differentiate only into hematopoietic tissues and those mesenchymal stem cells which are known to differentiate only into such paraxial mesodermal tissues as the skeletal muscle, adipocyte and bone, the above-mentioned mesenchymal cells have totipotency, namely they can differentiate into all the three germ layers, i.e. ectodermal, mesodermal and endodermal tissues.

Further, the present inventors analyzed the above-mentioned mesenchymal cells as to the expression of surface antigens using antibodies recognizing CD34, CD117, CD14, CD45, CD90, Sca-1, Ly6c and Ly6g, which are surface antigens of hematopoietic cells, antibodies recognizing Flk-1, CD31, CD105 and CD144, which are surface antigens of angioendotherial cells, antibodies recognizing CD140, which is a surface antigen of mesenchymal cells, antibodies recognizing CD49b, CD49d, CD29 and CD41, which are surface antigens of integrin, antibodies recognizing CD54, CD102, CD106 and CD44, which are matrix receptors, and so forth and, as a result, found that said mesenchymal cells are cells capable of differentiating into pancreatic β cells showing a so-far unknown, new mode of expression. Based on these findings, the present invention has now been completed.

Thus, the present invention provides the following (1) - (29) :
(1) A method of forming pancreatic β cells from mesenchymal cells which comprises subjecting mammal-derived mesenchymal cells to at least one step selected from the following steps (a) to (e) :
   a) the step of proliferating mesenchymal cells obtained from a mammalian sample and capable of differentiating into pancreatic β cells;
   b) the step of selecting and isolating those mesenchymal cells which are capable of differentiating into pancreatic β cells from among mesenchymal cells obtained from a mammalian sample or from among the mesenchymal cells obtained in step a) using an antibody or antibodies capable of binding to a cell membrane antigen;
   c) the step of cultivating the mesenchymal cells capable of differentiating into pancreatic β cells as obtained in step a) or b) or cells including such mesenchymal cells in an adhesion molecule/extracellular matrix-coated reaction vessel which enables the cells to contact with the adhesion molecules/extracellular matrix;
   d) the step of cultivating the mesenchymal cells capable of differentiating into pancreatic β cells as obtained in step a), b) or c) or cells including such mesenchymal cells in contact with a pancreatic β cell-forming agent; and
   e) the step of selecting and separating the pancreatic β cells obtained in the step c) or d) using a gene specifically expressed in pancreatic β cells as a selective marker.
(2) A method of forming pancreatic β cells from mesenchymal cells as defined in above (1), wherein the mesenchymal cells are obtained from bone marrow, muscle, pancreas, liver, small intestine, large intestine, kidney, subcutaneous tissue, endometrium, blood, cord blood or placenta.
(3) A method of forming pancreatic β cells from mesenchymal cells as defined in above (1) or (2), wherein the selection of mesenchymal cells in step b) is carried out using a CD140-positive antibody.
(4) A method of forming pancreatic β cells from mesenchymal cells as defined in any of above (1) to (3), wherein, in step e), the gene specifically expressed in pancreatic β cells is the insulin gene.
(5) A method of forming pancreatic β cells from mesenchymal cells as defined in any of above (1) to (4), wherein, in step d), the pancreatic β cell-forming agent comprises at least one member selected from the group consisting of cytokines, physiologically active substances, transcription factors and adhesion molecules/extracellular matrices.
(6) A method of forming pancreatic β cells from mesenchymal cells as defined in above (5), wherein the cytokine selected comprises at least one member selected from the group consisting of hepatocyte growth factor (HGF), fibroblast growth factor (bFGF)/FGF-2, insulin, transferrin, heparin-binding EGF, gastrin, TGF-β, insulin-like growth factor (IGF-1), parathyroid hormone-related proteins (PTHrP), growth hormone, prolactin, placental lactogen, glucagon-like peptide-1, exendin-4 and KGF (keratinocyte growth factor).
(7) A method of forming pancreatic β cells from mesenchymal cells as defined in above (5), wherein the physiologically active substance selected comprises at least one member selected from the group consisting of nicotinamide, betacellulin, activin A, progesterone, putrescine and selenium.
(8) A method of forming pancreatic β cells from mesenchymal cells as defined in above (5), wherein the transcription factor selected comprises at least one member selected from the group consisting of PTF1a/PTF-P48, Isl-1, Pdx-1/IPF-1, Beta2/neuroD, ngn3, PAX-6, PAX-4, Hlxb-9, Nkx2.2, Nkx6.1, HNF1α, HNF1β and HNF4α.
(9) A method of forming pancreatic β cells from mesenchymal cells as defined in above (5), wherein the adhesion molecule/extracellular matrix selected comprises at least one member selected from the group consisting of gelatin, laminin, collagen, agarose, fibronectin and ornithine.
(10) A pancreatic β cell-forming agent for use in the method as defined in any of above (1) to (9) which comprises, as an active ingredient, at least one member selected from the group consisting of cytokines, physiologically active substances, transcription factors and adhesion molecules/extracellular matrices.
(11) A pancreatic β cell-forming agent as defined in above (10), wherein the cytokine selected comprises at least one member selected from the group consisting of hepatocyte growth factor (HGF), fibroblast growth factor (bFGF)/FGF-2, insulin, transferrin, heparin-binding EGF, gastrin, TGF-β, insulin-like growth factor (IGF-1), parathyroid hormone-related proteins (PTHrP), growth hormone, prolactin, placental lactogen, glucagon-like peptide-1, exendin-4 and KGF.
(12) A pancreatic β cell-forming agent as defined in above (10), wherein the physiologically active substance selected comprises at least one member selected from the group consisting of nicotinamide, betacellulin, activin A, progesterone, putrescine and selenium.
(13) A pancreatic β cell-forming agent as defined in above (10) , wherein the transcription factor selected comprises at least one member selected from the group consisting of PTF1a/PTF-P48, Isl-1, Pdx-1/IPF-1, Beta2/neuroD, ngn3, PAX-6, PAX-4, Hlxb-9, Nkx2.2, Nkx6.1, HNF1α, HNF1β and HNF4α.
(14) A pancreatic β cell-forming agent as defined in above (10), wherein the adhesion molecule/extracellular matrix selected comprises at least one member selected from the group consisting of gelatin, laminin, collagen, agarose, fibronectin and ornithine.
(15) A method of screening candidate substances promoting the formation of pancreatic β cells from mesenchymal cells which comprises causing the formation of pancreatic β cells from mesenchymal cells according to the method as defined in above (1) in the presence of each candidate substance and selecting a candidate substance showing a pancreatic β cell formation-promoting effect in comparison with pancreatic β cells formed in the absence of the candidate substance.
(16) A method of screening candidate substances promoting the formation of pancreatic β cells from mesenchymal cells as defined in above (15), wherein the mesenchymal cells are obtained from bone marrow, muscle, pancreas, liver, small intestine, large intestine, kidney, subcutaneous tissue, endometrium, blood, cord blood or placenta.
(17) A method of screening candidate substances promoting the formation of pancreatic β cells from mesenchymal cells as defined in above (15) or (16), wherein the selection of mesenchymal cells in step b) is carried out using a CD140-positive antibody.
(18) A method of screening candidate substances promoting the formation of pancreatic β cells from mesenchymal cells as defined in any of above (15) to (17), wherein, in step e), the gene specifically expressed in pancreatic β cells is the insulin gene.
(19) A method of screening candidate substances promoting the formation of pancreatic β cells from mesenchymal cells as defined in any of above (15) to (18), wherein, in step d), the pancreatic β cell-forming agent comprises at least one member selected from the group consisting of cytokines, physiologically active substances, transcription factors and adhesion molecules/extracellular matrices.
(20) A method of screening candidate substances promoting the formation of pancreatic β cells from mesenchymal cells as defined in above (19), wherein the cytokine selected comprises at least one member selected from the group consisting of hepatocyte growth factor (HGF), fibroblast growth factor (bFGF)/FGF-2, insulin, transferrin, heparin-binding EGF, gastrin, TGF-β, insulin-like growth factor (IGF-1), parathyroid hormone-related proteins (PTHrP), growth hormone, prolactin, placental lactogen, glucagon-like peptide-1, exendin-4 and KGF.
(21) A method of screening candidate substances promoting the formation of pancreatic β cells from mesenchymal cells as defined in above (19), wherein the physiologically active substance selected comprises at least one member selected from the group consisting of nicotinamide, betacellulin, activin A, progesterone, putrescine and selenium.
(22) A method of screening candidate substances promoting the formation of pancreatic β cells from mesenchymal cells as defined in above (19), wherein the transcription factor selected comprises at least one member selected from the group consisting of PTF1a/PTF-P48, Isl-1, Pdx-1/IPF-1, Beta2/neuroD, ngn3, PAX-6, PAX-4, Hlxb-9, Nkx2.2, Nkx6.1, HNF1α, HNF1β and HNF4α.
(23) A method of screening candidate compounds promoting the formation of pancreatic p cells from mesenchymal cells as defined in above (19), wherein the adhesion molecule/extracellular matrix selected comprises at least one member selected from the group consisting of gelatin, laminin, collagen, agarose, fibronectin and ornithine.
(24) A method of screening candidate substances promoting the formation of pancreatic β cells from mesenchymal cells as defined in any of above (15) to (23), wherein the candidate substance is a cultivation-derived composition.
(25) A substance capable of promoting the formation of pancreatic β cells from mesenchymal cells as obtained by the method of screening candidate compounds promoting the formation of pancreatic β cells from mesenchymal cells as defined in any of above (15) to (24).
(26) A method for treating an impaired glucose tolerance-due disease of a patient which comprises administering an effective amount of the pancreatic β cells obtained by the method as defined in any of above (1) to (9) to the patient.
(27) A therapeutic agent for an impaired glucose tolerance-due disease which comprises, as an active ingredient, the pancreatic β cells obtainable by the method as defined in any of above (1) to (9).
(28) A method of causing differentiation into insulin-secreting cells which comprises scattering cells capable of differentiating into insulin-secreting cells on the layer of mesenchymal cells as obtained by monolayer culture on a culture dish and carrying out the cocultivation thereof.
(29) A method of causing differentiation into insulin-secreting cells by cocultivation as defined in above (28), wherein the cells capable of differentiating into insulin-secreting cell comprise at least one member selected from the group consisting of embryonic stem cells, pancreatic stem cells, small intestinal epithelial stem cells, liver-derived stem cells and amniotic cells.

The method of forming pancreatic β cells from mesenchymal cells according to the invention can be carried out by starting with mammal-derived mesenchymal cells (including mesenchymal cells capable of differentiating into pancreatic β cells) and, basically, by causing said cells to differentiate into pancreatic β cells (step c or d) and then separating (collecting) the thus-obtained pancreatic β cells (step e), as mentioned above.

The starting mesenchymal cells in the above method can be proliferated in the conventional manner (step a), and the desired mesenchymal cells capable of differentiating into pancreatic β cells can be selected and isolated from the starting cells (step b).

The thus-obtained cells capable of differentiating into pancreatic β cells are induced to differentiate into the desired pancreatic β cells by cultivation in a adhesion molecule/extracellular matrix (pancreatic β cell forming agent)-coated reaction vessel (step c) or by cultivation in contact with a pancreatic β cell forming agent (cultivation in a pancreatic β cell forming agent-containing medium) (step d).

In a preferred embodiment of the method of the present invention, mesenchymal cells capable of differentiating into pancreatic β cells as obtained from a mammal-derived sample are proliferated (step a), those mesenchymal cells which are capable of differentiating into pancreatic β cells are selected and isolated from among the mesenchymal cells from among the mesenchymal cells obtained in step a) using an antibody or antibodies capable of binding to a membrane antigen (step b) , the mesenchymal cells capable of differentiating into pancreatic β cells as obtained in step b) or cells containing these are cultivated in a adhesion molecule/extracellular matrix-coated reaction vessel which enables said cells to contact with the adhesion molecule/extracellular matrix (step c), the mesenchymal cells capable of differentiating into pancreatic β cells as obtained in step c) or cells containing these are cultivated while contacting them with a pancreatic β cell forming agent (step d), and the pancreatic β cells obtained in step d) are selected and isolated using, as a selective marker, a gene specifically expressed in the pancreatic β cells (step e).

In another preferred embodiment of the method of the present invention, mesenchymal cells capable of differentiating into pancreatic β cells as obtained from a mammal-derived sample are proliferated (step a), and the thus-obtained mesenchymal cells capable of differentiating into pancreatic β cells or cells containing these are then cultivated in a adhesion molecule/extracellular matrix-coated reaction vessel which enables said cells to contact with the adhesion molecule/extracellular matrix (step c).

In a further preferred embodiment of the method of the present invention, mesenchymal cells capable of differentiating into pancreatic β cells as obtained from a mammal-derived sample are proliferated (step a), and the thus-obtained mesenchymal cells capable of differentiating into pancreatic β cells or cells containing these are cultivated while contacting them with a pancreatic β cell forming agent (step d).

The formation (differentiation induction) of pancreatic β cells is most preferably realized by employing the step c and the next step d.

In carrying out the method of the invention, the mammal-derived mesenchymal cells (including mesenchymal cells capable of differentiating into pancreatic β cells) can be isolated from such vital tissues as bone marrow, muscle, pancreas, liver, small intestine, large intestine, kidney, subcutaneous tissue, endometrium, blood and cord blood, or placenta, and are preferably isolated from bone marrow or cord blood.

The above mesenchymal cells are pluripotent stem cells and are potentially capable of differentiating into not only pancreatic β cells but also various other cells (all vital tissue cells).

As a result of expression type analysis of cell surface markers, it was established that those mesenchymal cells capable of differentiating into pancreatic β cells which can be favorably utilized in carrying out the method of the invention are positive for CD140. The CD140-positive cells further include CD34-positive and CD117-positive cells, and CD34-negative and CD117-positive cells. Among them, the following cells are particularly preferred:
(1) CD34-positive, CD117-positive, CD14-negative, CD45-negative, CD90-negative, Flk-1-negative, CD31-negative, CD105-negative, CD144-positive, CD140-positive, CD49b-negative, CD49d-negative, CD29-positive, CD54-negative, CD102-negative, CD106-nagetive and CD44-positive cells;
(2) CD34-positive, CD117-positive, CD14-negative, CD45-negative, CD90-negative, Flk-1-negative, CD31-negative, CD105-negative, CD144-negative, CD140-positive, CD49b-negative, CD49d-negative, CD29-positive, CD54-negative, CD102-negative, CD.106-nagetive and CD44-positive cells;
(3) CD34-negative, CD117-positive, CD14-negative, CD45-negative, CD90-negative, Flk-1-negative, CD31-negative, CD105-negative, CD144-positive, CD140-positive, CD49b-negative, CD49d-negative, CD29-positive, CD54-negative, CD102-negative, CD106-nagetive and CD44-positive cells; and
(4) CD34-negative, CD117-positive, CD14-negative, CD45-negative, CD90-negative, Flk-1-negative, CD31-negative, CD105-negative, CD144-negative, CD140-positive, CD49b-negative, CD49d-negative, CD29-positive, CD54-negative, CD102-negative, CD106-nagetive and CD44-positive cells.

As the vital tissues or the like which are to serve as the sources of the mesenchymal cells, there may be mentioned those derived from mammals, preferably the mouse, rat, guinea pig, hamster, rabbit, cat, dog, sheep, swine, cattle, goat, monkey and human. For therapeutic use in humans, human-derived ones are particularly preferred.

In the following, the method of forming pancreatic β cells from mesenchymal cells derived from a source animal (method of isolating pancreatic β cells) is described in detail step by step or operation by operation.

### 1. Separation of mesenchymal cells capable of differentiating into pancreatic β cells from a source animal

The cells capable of differentiating into pancreatic β cells can be isolated from such various vital tissues as mentioned above or from cord blood. Hereinafter, specific mention is made of the method of isolating bone marrow-derived mesenchymal cells capable of differentiating into pancreatic β cells from the human bone marrow taken as an example of the vital tissue.

### (1) Isolation of bone marrow-derived mesenchymal cells

Bone marrow puncture is carried out in the sternum or ilium. The internal cylinder is pulled out of the bone marrow needle, a heparin-containing syringe is mounted on the needle, and a necessary amount of bone marrow fluid is aspirated swiftly. On an average, 10 ml to 20 ml of bone marrow fluid is aspirated. Bone marrow-derived mesenchymal cells are recovered from the thus-obtained bone marrow fluid by centrifugation at 1,000 x g, and the bone marrow-derived mesenchymal cells are then washed with PBS (phosphate buffered saline). After two repetitions of this procedure, the cells are resuspended in a cell culture medium to give a bone marrow-derived mesenchymal cell suspension.

As for the method of isolating bone marrow-derived mesenchymal cells (bone marrow-derived interstitial cell) capable of differentiating into pancreatic β cells from the above bone marrow-derived mesenchymal cell suspension, a conventional method for removing other cells coexisting in the cell suspension prepared as mentioned above, for example blood corpuscular cells, hematopoietic stem cells and vascular stem cells, can be followed. For example, the method described in M. F. Pittenger et al., Science, 284, 143 (1999) can be employed. Specifically, this method comprises first layering the bone marrow-derived mesenchymal cell suspension on a Percoll layer having a density of 1.073 g/ml and isolating and recovering the cells at the interface by 30 minutes of centrifugation at 1,100 x g. An equal volume of a 9/10 dilution of Percoll as resulting from addition of 10 x PBS was added to the bone marrow-derived mesenchymal cell suspension and, after mixing up, the mixture is centrifuged at 20,000 x g for 30 minutes, and a fraction showing a density of 1.075 to 1.060 g/ml is recovered. Thus is obtained a bone marrow-derived mesenchymal cell mixture containing bone marrow-derived mesenchymal cells capable of differentiating into pancreatic β cells.

The bone marrow-derived mesenchymal cell mixture containing bone marrow-derived mesenchymal cells capable of differentiating into pancreatic β cells as obtained in the above manner is diluted so that only one cell may be placed in each well of 96-well culture plates, a large number of one cell-derived clones are thus prepared, the clones are treated utilizing that method of deriving the desired pancreatic β cells from bone marrow-derived mesenchymal cells capable of differentiating into pancreatic β cells which is described below, and those clones in which insulin-producing cells appear are selected. Thus are obtained the desired bone marrow-derived mesenchymal cells capable of differentiating into pancreatic β cells.

The method of obtaining bone marrow-derived mesenchymal cells capable of differentiating into pancreatic β cells from a rat or mouse or the like is not particularly restricted but, for example, the following procedure can be followed. Thus, a rat or mouse is sacrificed by cervical dislocation and, after disinfection, the skin of the femur and the quadriceps muscle of thigh are excised. The knee joint portion is cut with scissors to remove the joint, and the muscle on the back of the femur is removed. The hip joint is cut with scissors to remove the joint, and the femur is taken out. The muscle adhering to the femur is removed as far as possible with scissors, and both ends of the femur are then cut off with scissors. A needle having an appropriate size adapted to the size of the bone is mounted on a 2.5-ml syringe, the syringe is filled with about 1. 5 ml of a cell culture medium, such as α-MEM (α-modified MEM), DMEM (Dulbecco's modified MEM) or IMDM (Isocove's modified Dulbecco's medium), supplemented with 10% of FBS (fetal bovine serum) and, then, the tip of the needle is inserted into the section, on the knee joint side, of the femur. The culture fluid in the syringe is injected into the bone marrow to thereby push out bone marrow-derived mesenchymal cells through the section, on the hip joint side, of the femur. The bone marrow-derived mesenchymal cells obtained are suspended in the culture fluid by pipetting. It is possible to isolate rat- or mouse bone marrow-derived mesenchymal cells capable of differentiating into pancreatic β cells from the thus-obtained bone marrow fluid in the same manner as the above-mentioned method of isolating bone marrow-derived mesenchymal cells from a human bone marrow fluid.

### (2) Isolation of mesenchymal cells capable of differentiating into pancreatic β cells from a tissue other than the bone marrow

Cells capable of differentiating into pancreatic β cells can be obtained from a tissue other than the bone marrow by the method of separation using an antibody, which is to be described later herein under 12.

A preferred example of the tissue other than the bone marrow is the cord blood. Specifically, cells capable of differentiating into pancreatic β cells can be isolated from the cord blood by the following method.

Thus, the cord blood is collected from the umbilical cord, immediately followed by addition of heparin to a final concentration of 500 units/ml. After thorough mixing, cells are recovered from the cord blood by centrifugation and resuspended in a cell culture medium, such as α-MEM, DMEM or IMDM, supplemented with 10% of FBS. Cells capable of differentiating into pancreatic β cells can be separated from the cell suspension obtained utilizing an antibody, as described later herein.

### 2. Cultivation of mesenchymal cells capable of differentiating into pancreatic β cells

The cultivation of mesenchymal cells capable of differentiating into pancreatic β cells is carried out in an appropriate medium. Generally, media having known compositions (cf. e.g. Soshiki Baiyo no Gijutsu Kisohen (Techniques in Tissue Culture, Fundamentals Section), 3rd edition, Asakura Shoten, 1996) can be used as the medium. As for the culture conditions, any conditions under which cells can be cultivated may be used. Generally, a temperature of 33-37°C is preferred as the culture temperature. Preferably, the cultivation is carried out in an incubator filled with 5-10% of carbon dioxide gas.

The bone marrow-derived mesenchymal cells capable of differentiating into pancreatic β cells are preferably proliferated in a state such that they are adhering to a plastic-made ordinary culture dish for tissue culture. When the culture dish is almost all over covered with proliferating cells, the medium is removed, and a trypsin-EDTA solution is added to thereby suspend the cells. The suspended cells are washed with PBS or a cell culture medium and then 5- to 20-fold diluted with the cell culture medium, and the dilution is added to a fresh culture dish, followed by further subcultivation.

### 3. Formation of pancreatic β cells from mesenchymal cells capable of differentiating into pancreatic β cells

The cultivation of mesenchymal cells capable of differentiating into pancreatic β cells as described above under 2 results in proliferation of the mesenchymal cells and, at the same time, in the formation of pancreatic β cells by differentiation of the mesenchymal cells.

The formation of pancreatic β cells from mesenchymal cells capable of differentiating into pancreatic β cells is effected more preferably by utilizing, as a pancreatic β cell forming agent, (1) a factor being expressed in the fetal pancreatic β cell development region or a factor serving in the differentiation into pancreatic β cells in the fetal pancreatic β cell development stage and/or (2) the culture supernatant of cells capable of differentiating into pancreatic β cells or the culture supernatant of pancreatic β cells resulting from differentiation of said cells. In the case of the above (2), feeder cells or cells capable of differentiating into insulin-secreting cells may be cocultured together with mesenchymal cells capable of differentiating into pancreatic β cells to achieve the same effect.

As the factor being expressed in the fetal pancreatic β cell development region or the factor serving in the differentiation into pancreatic β cells in the fetal pancreatic β cell development stage as described above under (1), there may be mentioned cytokines, adhesion molecules/extracellular matrices, physiologically active substances, and transcription factors, among others. Generally, these pancreatic β cell forming agents are utilized by adding them to the culture system for cells capable of differentiating into pancreatic β cells respectively in an appropriate amount. The addition level is not limited to a particular level but may be properly determined according to the kind of pancreatic β cell forming agent; generally, it can be within the range of about 1 to 400 ng/ml of culture fluid.

The cytokine may be any one provided that it is effective in promoting the differentiation of cells capable of differentiating into pancreatic β cells into pancreatic β cells in the stage of development of pancreatic β cells. As specific preferred examples of the cytokine, there may be mentioned the hepatocyte growth factor (HGF; cf. e.g. Nature Medicine, Vol. 6, Number 3, March 2000, 278-282, V. K. Ramiya et al.; Diabetes, Vo. 48, 1999, 1013-1019, Beattie, G. M., et al.; Endocrinology, Vol. 137, No. 9, 3969-3976, Hirosato Mashima, et al.; The Journal of Biological Chemistry, Vol. 275, No. 2, 14 January 2000, 1226-1232, Adolfo Garcia-Ocana, et al.), basic fibroblast growth factor (bFGF) , FGF-2 (cf. e.g. Science, Vol. 284, 18 June 1999, 1998-2003, Joonil Jung et al.; Nature, Vol. 408, 14 December 2000, 864-868, Alan W. Hart et al.), FGF-1 (cf. e.g. Science, Vol. 284, 18 June 1999, 1998-2003, Joonil Jung et al.; Nature, Vol. 408, 14 December 2000, 864-868, Alan W. Hart et al.), insulin, transferrin, heparin-binding epidermal growth factor (heparin-binding EGF; cf. The Journal of Biological Chemistry, Vol. 272, No. 46, 14 November 1997, 29137-29143, Hideaki Kaneto et al.), EGF (cf. e.g. Development, Vol. 112, 1991, 855-861, Hiroyuki Nogawa and Yu Takahashi; Nature Medicine, Vol. 6, Number 3, March 2000, 278-282, V. K. Ramiya et al.), gastrin, TGF-β1 (cf. Development, Vol. 120, 1994, 3451-3462, Sanvito F. et al.), insulin-like growth factors (IGF-1 etc.), parathyroid hormone-related protein (PTHrP; cf. The Journal of Biological Chemistry, Vol. 271, No. 2, 12 January 1996, 1200-1208, Rupangi C. Vasavada et al.), growth hormone, prolactin, placental lactogen (cf. The Journal of Biological Chemistry, Vol. 275, No. 20, 19 May 2000, 15399-15406, Rupangi C. Vasavada et al.), glucagon-like peptide-1 (cf. e.g. Diabetes, Vol. 49, May 2000, 741-748, Doris A. Stoffers et al.; Diabetes, Vol. 50, April 2001, 785-796, Hongxiang Hui et al.), exendin-4 (cf. e.g. Diabetes, Vol. 48, December 1999, 2270-2276, Gang Xu et al.; Diabetes, Vol. 49, May 2000, 741-748, Doris A. Stoffers et al.), keratinocyte growth factor (KGF; cf. e.g. PNAS, Vol. 97, No. 14, 5 July 2000, 7999-8004, Susan Bonner-Weir et al.; Am. J. Pathol., Vol. 145, 1994, 80-85, Yi. E. et al.), and TGAα (Development, Vol. 112, 1991, 855-861, Hiroyuki Nogawa and Yu Takahashi), among others.

It is also possible to promote the differentiation of cells capable of differentiating into pancreatic β cells into pancreatic β cells in the stage of development of pancreatic β cells by using an inhibitor of a cytokine inhibiting the differentiation into pancreatic β cells. As such inhibitory cytokine, there may be mentioned the fibroblast growth factor 2 (FGF-2), for instance. As its inhibitor, there may be mentioned an antibody or low-molecular compound neutralizing said cytokine, for instance.

Utilizable as the adhesion molecules/extracellular matrices are various adhesion molecules/extracellular matrices being expressed in the pancreatic β cell development region in the stage of development of pancreatic β cells. As specific examples thereof, there may be mentioned extracellular matrix proteins such as gelatin, laminin (Diabetes, Vol. 49, June 2000, 936-944, Christopher A. Crisera et al.; Diabetes, Vol. 48, April 1999, 722-730, Fang-Xu Jiang et al.; J. Anat., Vol. 193, 1998, 1-21, Monique Aumailley and Neil Smyth), collagen (Diabetes, Vol. 45, August 1996, 1108-1114, Julie Kerr-Conte et al.; Cell Tissue Res., Vol. 277, 1994, 115-121, Deijnen J. H. van et al. ; J. Tissue Cult. Method, Vol. 8, 1983, 31-36, Richards J. et al.), agarose, fibronectin, ornithine and the like, among others. When laminin is used, for instance, cells capable of differentiating into pancreatic β cells are cultured in a culture dish coated with laminin, whereby the differentiation of said cells into pancreatic β cells can be promoted.

As the physiologically active substance, there may be mentioned substances effective in promoting the differentiation into pancreatic β cells in the stage of development of pancreatic β cells such as nicotinamide (cf. Nature Medicine, Vol. 6, Number 3, March 2000, 278-282, V. K. Ramiya et al.; Transplantation, Vol. 68, No. 11, 15 December 1999, 1674-1683, Timo Otonkoski et al.), betacellulin (cf. Diabetes, Vol. 48, February 1999, 304-309, Hirosato Mashima et al.), activin A, progesterone, putrescine, selenium, B27-supplemented neurobasal (Journal of Neuroscience Research, Vol. 35, 1993, 567-576, G. J. Brewer et al.) and the like. Nicotinamide, for instance, is used at a concentration of 1 to 10 mM. Others, too, can be used at respective concentrations sufficient to produce the desired differentiation-promoting effect.

As the transcription factor, there may be mentioned PTF1a/PTF-P48 (Nature Genetics, Vol. 32, September 2002, 128-134), Isl-1 (Nature, Vol. 385, 16 January 1997, 257-260, Ulf Ahlgren et al.), Pdx-1/IPF-1 (Nature, Vol. 371, 13 October 1994, 606-609, Joorgen Jonsson et al.; Nature Genetics, Vol. 15, January 1997, 106-110, Doris A. Stoffers et al.; Nature, Vol. 408, 14 December 2000, 864-868, Alan W. Hart el al.), Beta2/neuroD (Genes & Development, Vol. 11, 1997, 2323-2334, Francisco J. Naya et al.), ngn3 (Development, Vol. 127, 3533-3542, 2000, Valeire M. Schwitzgebel et al.), PAX-6 (Nature, Vol. 387, 22 May 1997, 406-409, Luc St-Onge et al.), PAX-4 (Molecular and Cellular Biology, Vol. 19, No. 12, December 1999, 8281-8291, Yoshio Fujitani et al.; Molecular and Cellular Biology, Vol. 19, No. 12, December 1999, 8272-8280, Stuart B. Smith et al.), Hlxb-9 (Nature Genetics, Vol. 23, September 1999, 67-70, Hao Li et al.; Nature Genetics, Vol. 23, September 1999, 71-75, Kathleen A. Harrison et al.), Nkx2.2 (Development, Vol. 125, 2213-2221, 1998, L. Sussel et al.), Nkx6.1 (J. Histochem. Cytochem., Vol. 46, 1998, 707-715, Oster A. Jensen et al.), HNF1α, HNF1β and HNF4α (Nature Cell Biology, Vol. 2, December 2000, 879-887, Chia-Ning Shen et al.), cyclopamine, HNF3β, and HNF6, among others.

The above-mentioned transcription factors are introduced, in the form of DNAs coding for said factors, into cells capable of differentiating into pancreatic β cells and the DNAs are caused to be expressed so that the differentiation into pancreatic β cells can be induced. The introduction of DNAs coding for such transcription factors into cells and the expression thereof can be realized in the conventional manner.

The extracellular matrix obtained from pancreatic β cells can also be used as the pancreatic β cell forming agent. Since the extracellular matrix occurs in the culture supernatant of cells capable of differentiating into pancreatic β cells or in the culture supernatant of pancreatic β cells resulting from the differentiation of said cells, pancreatic β cells can also be derived from cells capable of differentiating into pancreatic β cells by utilizing such culture supernatants.

For example, when the desired cells are cultured on a cell culture dish coated with the pancreatic β cell-derived extracellular matrix, or when the desired cells are cocultured with pancreatic β cells forming that matrix, or when a culture supernatant containing that matrix is added to a culture medium and the desired cells are cultured therein, the differentiation of cells capable of differentiating into pancreatic β cells can be induced.

Further, the pancreatic β cell differentiation inducing factor obtainable by the method described below under 4 can also be utilized as a pancreatic β cell forming agent, and the utilization of the same, too, can induce the differentiation, into pancreatic β cells, of cells capable of differentiating into pancreatic β cells.

It is also possible to induce the differentiation into pancreatic β cells by cocultivating mesenchymal cells capable of differentiating into pancreatic β cells together with feeder cells. The term "feeder cells" as used herein means those cells which are cultured beforehand in a culture dish in the manner of monolayer culture in the case of induction of the differentiation into pancreatic p cells of the desired cells sown on the monolayer cells cultured in advance. As the feeder cells, there may be mentioned, for example, α, β, δ, PP cells (pancreatic polypeptide cells) constituting pancreatic islets of Langerhans, or pancreatic β cell-derived cell lines, or insulin-secreting cells such as insulinoma-derived cell lines. The cultivation of the feeder cells can be carried out in the same manner as the cultivation of cells capable of differentiating into pancreatic β cells, as described above 2.

Specifically, the cocultivation can be carried out by sowing mesenchymal cells capable of differentiating into pancreatic β cells over the monolayer feeder cells cultured beforehand in a culture dish. Further, in cocultivating mesenchymal cells capable of differentiating into pancreatic β cells together with feeder cells, it is also possible to carry out the cocultivation of the mesenchymal cells capable of differentiating into pancreatic β cells after fixation of the monolayer feeder cells cultivated in a culture dish with a fixer, such as paraformaldehyde, ethanol, or a 10% neutral buffered formalin solution.

In the above cocultivation, cells capable of differentiating into insulin-secreting cells can be used in lieu of the feeder cells. As examples of the cells capable of differentiating into insulin-secreting cells, there may be mentioned embryonic stem cells, pancreatic stem cells, small intestinal epithelial stem cells, liver-derived stem cells, and amniotic cells.

It is also possible to cause the differentiation into insulin-secreting cells by sowing cells capable of differentiating into insulin-secreting cells on mesenchymal cells cultivated in the manner of monolayer culture in a culture dish, followed by cocultivation. As the cells capable of differentiating into insulin-secreting cells, there may be mentioned embryonic stem cells, pancreatic stem cells, small intestinal epithelial stem cells, liver-derived stem cells, and amniotic cells.

### 4. Obtaining a factor inducing the differentiation into pancreatic β cells

As regards the method of obtaining a pancreatic β cell differentiation-inducing factor, such factor can be obtained by adding one of various protease inhibitors to the culture supernatant of insulin-producing cells and carrying out a procedure comprising a combination of dialysis, salting out, chromatography, etc.

Further, a gene for the factor inducing the differentiation into pancreatic β cells can be obtained by determining a partial amino acid sequence of the pancreatic β cell differentiation-inducing factor using a microsequencer, and screening a cDNA library constructed from said cells using a DNA probe designed based on said amino acid sequence.

The differentiation, into pancreatic β cells, of cells capable of differentiating into pancreatic β cells can be induced by utilizing the thus-obtained pancreatic β cell differentiation-inducing factor. For example, the desired pancreatic β cells can be obtained by adding that factor to a culture medium for cells capable of differentiating into pancreatic β cells and cultivating the cells capable of differentiating into pancreatic β cells. The cultivation conditions are as described above under 2. Generally, the addition level to the medium can be properly selected within the range of about 1 to 400 ng/ml.

### 5. Pancreatic β cell-regenerating agent or therapeutic agent for impaired glucose tolerance-due diseases, such as diabetes, which contains pancreatic β cells

The pancreatic β cells or precursors thereof resulting from induced differentiation according to the present invention are useful as a pancreatic β cell-regenerating agent or as a therapeutic agent for impaired glucose tolerance-due diseases, for example diseases resulting from impaired glucose tolerance, such as diabetes and like impaired glucose tolerance, and like various other diseases.

The pancreatic β cell-regenerating agent or therapeutic agent for impaired glucose tolerance-due diseases is required only to comprise highly pure pancreatic β cells or precursors thereof (cells capable of differentiating into pancreatic β cells) and, according to the site and size of impaired pancreatic β cells in a patient to which the agent is to be applied, use is made of the product of proliferation of said cells, preferably the one containing pancreatic β cell-like cells resulting from differentiation of cells capable of differentiating into pancreatic β cells and capable of producing insulin.

The cells of the invention which are to serve as the active ingredient of the pancreatic β cell-regenerating agent can be produced and purified, for example, from a bone marrow fluid of the patient by the density gradient centrifugation method described above under 1-(1), by the panning method using an antibody specifically recognizing cells capable of differentiating into pancreatic β cells as illustrated later herein under 8 [J. Immunol., 141 (8), 2797-2800 (1988)] or the FACSmethod [Int. Immunol., 10(3), 275-283 (1998)], or by the method comprising constructing a reporter system using a promoter of a gene specific to cells capable of differentiating into pancreatic β cells.

The cells which are an active ingredient of the agent mentioned above include the cells resulting from induced differentiation, into pancreatic β cells, of cells capable of differentiating into pancreatic β cells using a pancreatic β cell forming agent to be described below under 6, and the cells capable of differentiating into pancreatic β cells as resulting from application, to bone marrow-derived mesenchymal cells collected from a person of advanced aged, of the immortalization method described later herein under 11 to thereby activate the cell division activity.

The pancreatic β cell-regenerating agent or therapeutic agent for impaired glucose tolerance-due diseases according to the invention can be generally administered to patients requiring treatment at a dose within the range of about 10⁵-10⁸ cells/kg body weight, preferably about 10⁶-10⁷ cells/kg body weight. The route of administration is not particularly restricted but the method of delivery to the site of lesion by intraportal injection is preferred.

### 6. Pancreatic β cell forming agent

The pancreatic β cell forming agent of the invention comprises, as an active ingredient, at least one of factors being expressed in the fetal pancreatic β cell development region or factors causing the differentiation into pancreatic β cells in the stage of fetal pancreatic β cell development and factors inducing the differentiation into pancreatic β cells and, by utilizing the same, it is possible to induce the differentiation, into pancreatic β cells, of cells capable of differentiating into pancreatic β cells.

As the pancreatic β cell forming agent, there may be mentioned, among others, those cytokines, adhesion molecules/extracellular matrices, physiologically active substances and transcription factors as mentioned hereinabove in detail under 3.

Also utilizable as the pancreatic β cell forming agent are such feeder cells as those α, β, δ, PP cells (pancreatic polypeptide cells) constituting pancreatic islets of Langerhans, or pancreatic β cell-derived cell lines, or insulin-secreting cells such as insulinoma-derived cell lines and the like as mentioned above under 2.2, and such cells capable of differentiating into insulin-secreting cells as embryonic stem cells, pancreatic stem cells, small intestinal epithelial stem cells, liver-derived stem cells and amniotic cells.

These can be used generally at a concentration within the range of about 1-400 ng/ml. Preferably, the cytokines are used at a concentration of 10-40 ng/ml. Nicotinamide as a physiologically active substance is used at a concentration of 10⁻³ M, for instance. As for the adhesion molecules/extracellular matrices, in the case of laminin, for instance, a culture dish coated therewith is utilized and cells capable of differentiating into pancreatic β cells are cultured in the culture dish, whereby their differentiation into pancreatic β cells can be promoted.

The pancreatic β cell forming agent of the present invention includes the pancreatic β cell differentiation inducing factors or those comprising genes for the pancreatic β cell differentiation inducing factors as described above under 4. In the following, these are described in detail.

### (1) Pancreatic β cell forming agent comprising a gene coding for a pancreatic β cell differentiation inducing factor

The pancreatic β cell forming agent comprising a gene coding for a pancreatic β cell differentiation inducing factor as an active ingredient is described in detail.

First, a pancreatic β cell differentiation inducing factor gene DNA fragment or the full-length cDNA thereof is inserted into a viral vector plasmid at a site downstream from a promoter to construct a recombinant viral vector plasmid. Utilizable here as the viral vector are retrovirus vectors, lentivirus vectors, adenovirus vectors, adeno-associated virus vectors and various other known ones.

Then, the recombinant viral vector plasmid is introduced into packaging cells adapted to the viral vector plasmid. The recombinant virus vector plasmid lacks at least one of the genes encoding the proteins necessary for the packaging of a virus. As the packaging cell, any cell can be used so far as it can supply the protein encoded by the lacking gene. For example, human kidney-derived HEK293 cells, mouse fibroblast NIH3T3 cells and the like can be used as the packaging cells.

Usable as the protein to be supplied by the packaging cells are such murine retrovirus-derived proteins as gag, pol and env in the case of retrovirus vectors, such HIV virus-derived proteins as gag, pol, env, vpr, vpu, vif, tat, rev and nef in the case of lentivirus vectors, such adenovirus-derived proteins as E1A and E1B in the case of adenovirus vectors, and such proteins as Rep(p5,p19,p40) and Vp(Cap) in the case of adeno-associated virus vectors.

To be used as the viral vector plasmid are those capable of producing a recombinant virus in the packaging cells mentioned above and containing a promoter at a site allowing the transcription, in pancreatic β cells, of a wild-type gene corresponding to the MODY-causing gene. Specific examples thereof are such viral vector plasmids as MFG [Proc. Natl. Acad. Sci. USA, 92, 6733-6737 (1995)], pBabePuro [Nucleic Acids Research, 18, 3587-3596 (1990)], LL-CG, CL-CG, CS-CG, CLG [Journal of Virology, 72, 8150-8157 (1998)], and pAdex1 [Nucleic Acids Res., 23, 3816-3821 (1995)], among others.

Any promoter that can be expressed in human tissues can be used as the promoter. Thus, mention may be made of the cytomegalovirus (human CMV) IE (immediate early) gene promoter, SV40 early promoter, retrovirus promoter, metallothionein promoter, heat shock protein promoter, and SRα promoter, for instance. The human CMV IE gene enhancer can be used together with the above promoter. Further, when a promoter of a gene specific in a pancreatic β cell such as insulin gene is utilized, the expression of the desired gene can be caused specifically in pancreatic β cells.

As the method of introducing the above-mentioned recombinant viral vector plasmid into the packaging cells mentioned above (method of recombinant viral vector production), there may be mentioned, for example, the lipofection method [Proc. Natl. Acad. Sci. USA, 84, 7413 (1987)], among others.

A pancreatic β cell forming agent can be prepared by formulating the thus-obtained recombinant viral vector together with a base used in gene therapy agent. The preparation can be carried out following the method described in the literature [Nature Genet., 8, 42 (1994)]. The base to be used in gene therapy agent may be any base generally used in injections. Mention may be made, for example, distilled water, salt solutions containing sodium chloride or a mixture of sodium chloride and another or other inorganic salts, solutions of mannitol, lactose, dextran, glucose, etc., solutions of amino acids such as glycine, arginine, etc., and mixed solutions composed of an organic acid solution or a salt solution and a glucose solution. It is also possible to prepare injections in the form of solutions, suspensions or dispersions in the conventional manner using, together with such bases, one or more auxiliaries selected from among osmotic pressure modifiers, pH adjusting agents, vegetable oils such as sesame oil and soybean oil, lecithin, surfactants such as nonionic surfactants, etc. It is also possible, by powder formulation, lyophilization or a like process, to prepare these injections in the form of preparations to be dissolved before each use. The thus-obtained pancreatic β cell forming agent can be used in gene therapy as it is when it occurs as a liquid or, when it occurs as a solid, after dissolving, just prior to treatment, in the above-mentioned base, if necessary following sterilization thereof. The pancreatic β cell forming agent can be practically used in gene therapy by the method comprising locally administering using a catheter and the like.

It is also possible to perform the gene therapy according to the invention by administering, to a patient, those infected cells obtained by infecting cells capable of differentiating into pancreatic β cells with the above-mentioned recombinant viral vector in a test tube as the pancreatic β cell forming agent. The desired gene therapy can also be given to a patient by administering the above-mentioned recombinant viral vector directly to the infected site of the patient.

### (2) Pancreatic β cell forming agent comprising a protein as an active ingredient

In the following, the pancreatic β cell forming agent comprising a pancreatic β cell differentiation inducing factor as an active ingredient is described in detail.

Based on the full-length cDNA for the pancreatic β cell differentiation inducing factor, a DNA fragment having an appropriate length and containing the portion coding for that protein is prepared where necessary. That DNA fragment or the full-length cDNA is inserted into an expression vector at a site downstream from a promoter to construct a recombinant expression vector for that protein. The recombinant expression vector is introduced into host cells adapted to the expression vector.

Cells that can allow the expression of the desired DNA can all be used as the host cells. Usable, for example, are bacterial cells belonging to the genera Escherichia, Serratia, Corynebacterium, Brevibacterium, Pseudomonas, Bacillus, Microbacterium, etc., yeast cells belonging to the genera Kluyveromyces, Saccharomyces, Schizosaccharomyces, Trichosporon, Schwanniomyces, etc., animal cells, and insect cells, among others.

As specific examples of the host cells, there may be mentioned, for example, Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Bacillus subtilis, Bacillus amyloliquefaciens, Brevibacterium ammoniagenes, Brevibacterium immariophilum ATCC 14068, Brevibacterium saccharolyticum ATCC 14066, Corynebacterium glutamicum ATCC 13032, Corynebacterium glutamicum ATCC 14067, Corynebacterium glutamicum ATCC 13869, Corynebacterium acetoacidophilum ATCC 13870, Microbacterium ammoniaphilum ATCC 15354, Pseudomonas sp. D-0110, etc.

The expression vector that can be employed are those capable of autonomously replicating or being incorporated into a chromosome in the host cells and containing a promoter at a site allowing the transcription of the pancreatic β cell differentiation inducing factor gene DNA.

In cases where bacterial cells are used as the host cells, it is preferable that the recombinant expression vector of the pancreatic β cell differentiation inducing factor is capable of autonomously replicating in the bacterial cells and, at the same time, is a recombinant expression vector comprising the promoter, ribosome-binding sequence, pancreatic β cell differentiation inducing factor-encoding DNA and transcription termination sequence. The vector can further comprise a gene regulating the promoter.

As such expression vector, there may be mentioned, for example, pBTrp2, pBTac1, pBTac2 (all available from Boehringer Mannheim), pKK233-2 (product of Amersham Pharmacia Biotech), pSE280 (product of Invitrogen), pGEMEX-1 (product of Promega), pQE-8 (product of Qiagen), pKYP10 [Japanese Published Unexamined Patent Application No.1983-110600], pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (product of Stratagene), pGEX (product of Amersham Pharmacia Biotech), pET-3 (product of Novagen), pTerm2 (USP 4686191, USP 4939094, USP 5160735), pSupex, pUB110, pTP5, pC194, pEG400 [J. Bacteriol., 172, 2392 (1990)], etc.

The expression vector to be used is preferably one in which the distance between the Shine-Dalgarno sequence, which is the ribosome-binding sequence, and the initiation codon is properly adjusted (e.g. to 6 to 18 bases).

The promoter may be any one provided that it can be expressed in the host cells. For example, mention may be made of Escherichia coli- or phage-derived promoters such as the trp promoter (Ptrp), lac promoter (Plac), P_{L} promoter, P_{R} promoter and T7 promoter as well as the SP01 promoter, SP02 promoter, and penP promoter. Artificially modified promoters such as the promoter (PtrpX2) resulting from connection of two Ptrps in tandem, the tac promoter, letI promoter [Gene, 44, 29 (1986)] and lacT7 promoter can also be used.

One or more codons in the base sequence of the pancreatic β cell differentiating inducing factor-encoding portion can be replaced with one or ones best suited for the expression in the host to thereby improve the productivity of the desired protein.

The transcription termination sequence is not essential for the expression of the pancreatic β cell differentiation inducing factor but it is desirable that the transcription termination sequence be positioned preferably just downstream from the structural gene.

As for the method of introducing the recombinant vector, any method of introducing DNA into host cells can be used. For example, mention may be made of the method using the calcium ion [Proc. Natl. Acad. Sci. USA, 69, 2110 (1972)], and the methods described in Gene, 17, 107 (1982) and Molecular & General Genetics, 168, 111 (1979).

As the expression vector to be used when yeast cells are used as the host cells, there may be mentioned YEp13 (ATCC 37115), YEp24 (ATCC 37051), YCp50 (ATCC 37419), pHS19, and pHS15, for instance.

The promoter may be any one that can be expressed in yeasts. For example, mention may be made of the PH05 promoter, PGK promoter, GAP promoter, ADH promoter, gall promoter, gal10 promoter, heat shock protein promoter, MFα1 promoter, and CUP1 promoter.

As the host cells, there may be mentioned Saccharomyces cerevisiae, Schizosaccharomyces pombe, Kluyveromyces lactis, Trichosporon pullulans and Schwanniomyces alluvius, among others.

As for the method of introducing the recombinant vector, any method that can serve to introduce DNA into yeasts can be used. For example, mention may be made of the electroporation method [Methods in Enzymol., 194, 182 (1990)], spheroplast method [Proc. Natl. Acad. Sci. USA, 75, 1929 (1978)], and lithium acetate method [J. Bacteriol., 153, 163 (1983; Proc. Natl. Acad. Sci. USA, 75, 1929 (1978)), among others.

As examples of the expression vector to be used when animal cells are used as the host cells, there may be mentioned, among others, pCDNAI (product of Invitrogen), pCDM8 (product of Invitrogen), pAGE107 [Cytotechnology, 3, 133 (1990)], pCDM8 [Nature, 329, 840 (1987)], pCDNAI/Amp (product of Invitrogen), pREP4 (product of Invitrogen), pAGE103 [J. Biochem., 101, 1307 (1987)], and pAGE210.

As for the promoter, any one that can be expressed in animal cells can be used. For example, mentioned may be made of the cytomegalovirus (human CMV) IE (immediate early) gene promoter, SV40 early promoter, retrovirus promoter, metallothionein promoter, heat shock protein promoter, and SRα promoter. The human CMV IE gene enhancer may be used together with the promoter.

Usable as the host cells are Namalwa cells, which are human cells, COS cells, which are simian cells, and CHO cells, which are Chinese hamster cells, among others.

As for the method of introducing the recombinant vector, any method that can serve to introduce DNA into animal cells can be used. For example, use may be made of the electroporation method [Cytotechnology, 3, 133 (1990)], andlipofectionmethod [Proc. Natl. Acad. Sci. USA, 84, 7413 (1987); Virology, 52, 456 (1973)], among others.

When insect cells are used as the host, protein expression can be realized by the methods described in Baculovirus Expression Vectors, A Laboratory Manual, W. H. Freeman and Company, New York (1992), Current Protocols in Molecular Biology, Supplement, 1-38 (1987-1997), and Bio/Technology, 6, 47 (1988), for instance.

Thus, the recombinant gene transfer vector and baculovirus are cointroduced into insect cells to give the desired recombinant virus in the insect cell culture supernatant, and insect cells are infected with the recombinant virus, whereby protein expression can be realized.

As the gene transfer vector to be used in the above methods, there may be mentioned pVL1392, pVL1393, and pBlueBacIII (all being products of Invitrogen), among others.

Autographa californica nuclear polyhedrosis virus, which is a virus infecting insects of the family Barathra, for instance, can be used as the baculovirus.

Employable as the insect cells are Sf9 and Sf21 [Baculovirus Expression Vectors, A Laboratory Manual, W. H. Freeman and Company, New York (1992), which are ovary cells of Spodoptera frugiperda, and High 5 (product of Invitrogen), which is ovary cells of Trichoplusia ni, among others.

As the method of cointroducing the above-mentioned recombinant gene transfer vector and baculovirus into insect cells for recombinant virus preparation, there may be mentioned, for example, the lipofection method [Proc. Natl. Acad. Sci. USA, 84, 7413 (1987)].

Expression of the gene can be carried out not only by direct expression but also by secretory production, fused protein expression, etc. according to the methods described in Molecular Cloning, A Laboratory Manual, Second Edition, Cold Spring Harbor Laboratory Press (1989) (hereinafter referred to as "Molecular Cloning, A Laboratory Manual, 2nd ed.") etc.

In the case of expression in yeasts, animal cells or insect cells, proteins resulting from addition of a sugar or sugar chain can be obtained.

The pancreatic β cell differentiation inducing factor can be produced by cultivating a transformant carrying the recombinant DNA with the pancreatic β cell differentiation inducing factor-encoding DNA inserted therein in a medium to thereby cause formation and accumulation of the pancreatic β cell differentiation inducing factor in the culture and recovering the protein from the culture.

The cultivation of the pancreatic β cell differentiation inducing factor-producing transformant in a medium can be carried out according to any of the conventional methods generally used in host cultivation.

The medium for cultivating the transformants obtained by using prokaryotes such as Escherichia coli or eukaryotes such as yeasts as the host cell may be a natural medium or synthetic medium which contains a carbon source (s), nitrogen source (s), inorganic substance (s) and one or more other substances assimilable by the host and in which the cultivation of the transformant can be carried out efficiently.

The carbon source(s) may be one(s) assimilable by the respective hosts. Utilizable as such are carbohydrates such as glucose, fructose, sucrose, molasses containing these, starch and starch hydrolyzates, organic acids such as acetic acid and propionic acid, and alcohols such as ethanol and propanol.

Utilizable as the nitrogen sources are ammonia, various inorganic acid or organic acid ammonium salts such as ammonium chloride, ammonium sulfate, ammonium acetate and ammonium phosphate, other nitrogen-containing compounds, and peptone, meat extracts, yeast extract, casein hydrolyzates, soybean cake, soybean cake hydrolyzates, various cells resulting from fermentation, digests thereof, etc.

Utilizable as the inorganic substances are monopotassium phosphate, dipotassium phosphate, magnesium phosphate, magnesium sulfate, sodium chloride, ferrous sulfate, manganese sulfate, copper sulfate, calcium carbonate, etc.

The cultivation is carried out under aerobic conditions in the manner of shake culture or submerged culture with aeration and stirring, for instance. The cultivation temperature is preferably 15-40°C and, generally, the cultivation time may be 16 hours to 7 days. During cultivation, the pH is preferably maintained at 3.0 to 9.0. The pH adjustment can be made using an inorganic or organic acid, an alkali solution, urea, calcium carbonate, or ammonia, for instance. If necessary, antibiotics such as ampicillin or tetracycline may be added to the medium during cultivation.

In cultivating a microorganism transformed with an expression vector in which an inducible promoter is used as the promoter, an appropriate inducer may be added to the medium according to need. Thus, in cultivating a microorganism transformed with an expression vector in which the lac promoter is used, isopropyl β-D-thiogalactopyranoside (IPTG), for instance, may be added to the medium and, in cultivating a microorganism transformed with an expression vector in which the trp promoter is used, indoleacrylic acid (IAA), for instance, may be added to the medium.

Usable as the medium for cultivating transformants obtained by using animal cells as the host cells are those in general use, such as RPMI 1640 medium [The Journal of the American Medical Association, 199, 519 (1967)], Eagle's MEM [Science, 122, 501 (1952)], Dulbecco's modified MEM [Virology, 8, 396 (1959)], 199 medium [Proceedings of the Society for the Biological Medicine, 73, 1 (1950)], and media derived from these media by supplementing fetal calf serum.

The cultivation can be carried out generally under the conditions of pH 6-8, 30-40°C, in the presence of 5% CO₂, for 1-7 days. During cultivation, antibiotics such as kanamycin or penicillin may be added to the medium according to need.

Employable as the medium for cultivating transformants obtained by using insect cells as the host cells are those in general use, such as TNM-FH medium (product of Pharmingen), Sf-900 II SFM medium (product of Life Technologies), ExCel1400 and ExCel1405 (both being products of JRH Biosciences), Grace's Insect Medium [Grace, T.C.C., Nature, 195, 788 (1962)], etc.

The cultivation can be carried out generally under the conditions of pH 6-7, 25-30°C, for 1-5 days.

If necessary, antibiotics such as gentamicin may be added to the medium during cultivation.

For isolating and purifying the pancreatic β cell differentiation inducing factor from the above-mentioned transformant culture, ordinary methods of isolating and purifying proteins may be used. For example, the pancreatic β cell differentiation inducing factor is expressed in a state dissolved in cells, the cells are recovered, after completion of cultivation, by centrifugation, then suspended in an aqueous buffer solution, and disrupted using a sonicator or the like, whereby a cell-free extract is obtained. The cell-free extract is centrifuged. From the supernatant thus obtained, a purified preparation can be obtained by an ordinary method of protein isolation and purification, for example by the single use or combined use of such techniques as solvent extraction, salting out with ammonium sulfate, desalting, precipitation with an organic solvent, diethylaminoethyl (DEAE) -Sepharose, anion exchange chromatography using such a resin as Diaion HPA-75 (product of Mitsubishi Chemical) , cation exchange chromatography using such a resin as S-Sepharose FF (product of Amersham Pharmacia Biotech), hydrophobic chromatography using such a resin as butyl-Sepharose or phenyl-Sepharose, gel filtration using a molecular sieve, affinity chromatography, chromatofocusing, and electrophoresis such as isoelectric focusing.

In cases where the protein in question is expressed in an insolubilized form in cells, the cells are recovered, disrupted and subjected to centrifugation to recover the insoluble form of protein as a precipitate fraction. The thus-recovered insoluble form of protein is solubilized using a protein denaturing agent. The concentration of the protein denaturing agent in the solubilization liquid is lowered by dilution or dialysis to thereby bring the structure of the protein back to its normal steric structure and, then, a purified preparation of the protein is obtained by the same method of isolation and purification as mentioned above.

In cases where the pancreatic β cell differentiation inducing factor or a derivative thereof, such as glycosylated protein, is secreted out of cells, they can be recovered from the culture supernatant. For example, the culture supernatant is recovered from the culture by such a technique as centrifugation, and a purified preparation can be obtained from the culture supernatant using the same isolation/purification method as mentioned above.

The protein expressed by each of the methods mentioned above can also be separately produced by chemical synthesis such as the Fmoc process (fluorenylmethyloxycarbonyl process) or tBoc process (tert-butyloxycarbonyl process) based on the amino acid sequence thereof. It is also possible to synthesize the same utilizing a peptide synthesizer such as one available from Advanced ChemTech (U.S.A), Perkin-Elmer (U.S.A), Amersham Pharmacia Biotech (U.S.A.), Protein Technology Instrument (U.S.A.), Synthecell-Vega (U.S.A.), PerSeptive (U.S.A.) or Shimdzu Corporation, for instance.

A protein capable of inducing the differentiation into pancreatic β cells can be practically used as a pancreatic β cell forming agent in the same manner as described above under 6-(1).

### 7. Application to therapy of congenital genetic disease

Among impaired glucose tolerance-due diseases such as diabetes, there is a group of diseases in which impaired glucose tolerance results due to deficiency of a part of proteins necessary for the differentiation into or maintenance of pancreatic β cells as resulting from mutation in a single gene. Typical examples as such disease include MODY (maturity onset diabetes of the young). This disease is known to be due to a genetic abnormality in relation to myosin, troponin, tropomyosin, voltage-dependent Na channel, K channel, fibrin, elastin, mitochondria, dystrophin, etc.

As the method of treatment of patients with the above disease, there may be mentioned the method comprising collecting cells capable of differentiating into pancreatic β cells from the patient with the disease, introducing a normal gene into those cells, and then transplanting the cells onto the pancreas. The normal gene can be inserted into a vector for gene therapy as described above under 6-(1) and then introduced into cells capable of differentiating into pancreatic β cells.

### 8. Antibody recognizing a surface antigen specific to cells capable of differentiating into pancreatic β cells

Now, the antibody recognizing a surface antigen expressed on the cells capable of differentiating into pancreatic β cells according to the invention is described in detail.

The antibody recognizing a surface antigen specifically expressed on the cells capable of differentiating into pancreatic p cells according to the invention can be used in purity testing or purification of cells capable of differentiating into pancreatic β cells, which is necessary for carrying out cell therapy of impaired glucose tolerance-due diseases.

As for the method of obtaining said antibody, 3-5 x 10⁵ cells/animal of the cells capable of differentiating into pancreatic β cells according to the invention or about 1-10 mg/animal of a cell membrane fraction prepared from those cells are used as an antigen and administered to nonhuman mammals, such as rabbits, goats, 3-20 week old rats, mice, or hamsters, subcutaneously, intravenously or intraperitoneally, together with an appropriate adjuvant (e.g. complete Freund's adjuvant, aluminum hydroxide gel, pertussis vaccine).

The administration of the antigen is carried out 3 to 10 times at 1- to 2-week intervals after the first administration. On days 3 to 7 after each administration, blood is sampled from the eyeground venous plexus for testing as to whether the serum obtained reacts with the antigen used for immunization or not by an enzyme immunoassay method [ELISA, published 1976 by Igaku Shoin; Antibodies - A Laboratory Manual, Cold Spring Harbor Laboratory, 1988], for instance.

Those nonhuman mammals whose serum has showed a sufficient antibody titer against the antigen used for immunization are used as sources of supply of serum or antibody-producing cells.

Polyclonal antibodies can be prepared by separation and purification from the serum.

Monoclonal antibodies can be prepared by producing hybridomas by fusion between the antibody-producing cells and nonhuman mammal-derived myeloma cells, cultivating the hybridomas or administering them to animals to form ascites tumors in the animals, and separating and purifying antibodies from the cultures or ascitic fluids.

Splenocytes, lymphatic nodes, and antibody-producing cells in the peripheral blood are used as the antibody-producing cells, and splenocytes are particularly preferred.

Preferably used as the myeloma cells are the 8-azaguanine-resistant mouse (BALB/c-derived) myeloma cell lines P3-X63Ag8-U1 (P3-U1) [Current Topics in Microbiology and Immunology, 18, 1 (1978)], P3-NS1/1-Ag41 (NS-1) [European J. Immunology, 6, 511 (1976)], SP2/0-Ag14 (SP-2) [Nature, 276, 269 (1978)], P3-X63-Ag8653 (653) [J. Immunology, 123, 1548 (1979)], and P3-X63-Ag8 (X63) [Nature, 256, 495 (1975)], and like mouse-derived established cell lines.

The hybridoma cells can be produced by the following method. Thus, the antibody-producing cells and myeloma cells are mixed together, suspended in HAT medium (medium resulting from addition of hypoxanthine, thymidine and aminopterine to normal medium) and cultivated for 7 to 14 days. After cultivation, a part of the supernatant is collected, and cells reacting with the antigen but unreactive with an antigen-free protein are selected by enzyme immunoassay or like testing. Then, cloning is performed by the limiting dilution method, and cells stably showing a high antibody titer as determined by enzyme immunoassay are selected as monoclonal antibody-producing hybridoma cells.

As the method of isolating and purifying polyclonal antibodies or monoclonal antibodies, there may be mentioned the single or combined use of such techniques as centrifugation, ammonium sulfate precipitation, caprylic acid precipitation, and chromatographic techniques using a DEAE-Separose column, anion exchange column, protein A or G column, or gel filtration column.

Whether test cells are expressing a surface antigen or not can be easily judged by using the antibody recognizing the surface antigen being expressed in cells capable of differentiating into pancreatic β cells as obtained in the above manner and making a comparison between the reactivity thereof with the test cells and the reactivity thereof with control cells such as hematopoietic stem cells and nervous system stem cells.

### 9. Obtaining a surface antigen being expressed in cells capable of differentiating into pancreatic β cells and obtaining gene coding for that surface antigen

As the method of obtaining a surface antigen gene being specifically expressed in cells capable of differentiating into pancreatic β cells, there may be mentioned a method of obtaining a gene showing different modes of expression in two samples differing in origin, namely the subtraction method [Proc. Natl. Acad. Sci. USA, 85, 5738-5742 (1988)], or the method based on representational difference analysis [Nucleic Acids Research, 22, 5640-5648 (1994)).

First, a cDNA library constructed from cells capable of differentiating into pancreatic β cells is subjected to subtraction using mRNA obtained from control cells other than cells capable of differentiating into pancreatic β cells, for example from hematopoietic stem cells or nervous system stem cells. After preparation of a subtracted cDNA library in which genes specific to the cells capable of differentiating into pancreatic β cells are concentrated, the insert cDNA sequences in the subtracted cDNA library are subjected to random base sequence analysis from the 5' terminal side, and only those having a secretory signal sequence are selected (random sequence analysis). Base sequence determination of the full-length base sequence of the thus-obtained cDNA makes it possible to make distinction as to whether the protein encoded by the cDNA is a secretory protein or a membrane protein.

In the above process, signal sequence trapping [Science, 261, 600-603 (1993); Nature Biotechnology, 17, 487-490 (1999)] can also be used in lieu of random sequence analysis. The signal sequence trapping method consists in selectively screening for genes having a secretory signal sequence.

For obtaining a specific surface antigen efficiently, the method is desirable which comprises constructing a signal sequence trap library using a vector capable of effecting subtraction and subjecting the signal sequence trap library constructed from cells capable of differentiating into pancreatic β cells to subtraction using mRNA obtained from control cells such as hematopoietic stem cells or nervous system stem cells. The thus-obtained, secretory signal sequence-containing DNA fragment can be used as a probe for cloning the full-length cDNA.

The proteins encoded by the cDNAs can be distinguished into secretory proteins and membrane proteins by determining the full length nucleotide sequences of the full length cDNAs.

In either case of random sequence analysis or signal sequence trapping, when the clone obtained codes for a membrane protein, a synthetic peptide is produced based on the amino acid sequence deduced from the base sequence. Then, a specific antibody can be obtained by the above-mentioned method using that synthetic peptide as an antigen.

In the case of membrane proteins, some encodes receptors. Such receptors possibly serve to adjust the specific proliferation of cells capable of differentiating into pancreatic β cells or the differentiation thereof into pancreatic β cells, and they can be used in searching for ligands of said receptors. In the case of a secretory protein, it can be directly used for proliferating or differentiating cells capable of differentiating into pancreatic β cells.

### 10. Screening for candidate substances promoting pancreatic β cell formation

The present invention also provides a method of screening for candidate substances having pancreatic β cell formation (differentiation induction) promoting activity. The screening method can be carried out by adding a candidate substance to the culture system on the occasion of cultivating mesenchymal cells capable of differentiating into pancreatic β cells in a serum-free medium and checking whether the presence thereof promotes the pancreatic β cell formation or not, namely checking the level of pancreatic β cells resulting from differentiation induction.

The candidate substances include various cytokines, growth factors and other secretory proteins, cell adhesion molecules/extracellular matrices and other membrane-binding proteins, tissue extracts, synthetic peptides, synthetic compounds, microbial culture fluids, and so forth.

The proliferating ability of pancreatic β cells or precursors thereof can be checked in terms of colony forming ability or BrdU uptake, for instance.

The colony forming ability can be checked by sowing, at a low density, the cells capable of differentiating into pancreatic β cells according to the invention.

The BrdU uptake can be checked by immunostaining using an antibody specifically recognizing BrdU.

As the method of evaluating the differentiation into pancreatic β cells, there may be mentioned, among others, the method using the insulin production by cells as an indicator and the method using the expression of a reporter gene introduced into cells as an indicator.

The method using the reporter gene expression as an indicator comprises introducing a vector DNA with a promoter of a gene specifically expressed in pancreatic β cells and a reporter gene into cells capable of differentiating into pancreatic β cells and checking the expression of the reporter gene using those cells.

As the reporter gene, there may be mentioned the genes coding for GFP (green fluorescent protein), luciferase, and betagalactosidase.

As the promoter of a gene specifically expressed in pancreatic β cells, there may be mentioned insulin.

### 11. Immortalization of cells capable of differentiating into pancreatic β cells

In the case of administering the therapeutic agent of the invention to patients, in particular aged patients, with diabetes or some other impaired glucose tolerance-due disease, it is desirable that the frequency of cell division of the cells capable of differentiating into pancreatic β cells according to the invention be increased without allowing canceration.

As the method of increasing the frequency of cell division without allowing canceration, there may be mentioned the method comprising causing telomerase to be expressed in the cells capable of differentiating into pancreatic β cells according to the invention.

As the method of causing expression of telomerase in the cells capable of differentiating into pancreatic β cells according to the invention, there may be mentioned, among others, the method comprising introducing the TERT gene for a catalytic subunit of telomerase into a retrovirus vector and introducing the vector into cells capable of differentiating into pancreatic β cells, the method comprising administering a factor inducing the expression of the TERT gene intrinsic in cells capable of differentiating into pancreatic β cells to cells capable of differentiating into pancreatic β cells, and the method comprising introducing a vector containing a DNA coding for a factor inducing the expression of the TERT gene into cells capable of differentiating into pancreatic β cells.

The above-mentioned factor inducing the expression of the TERT gene can be selected by introducing a vector DNA with the TERT gene promoter and a reporter gene such as GFP (green fluorescent protein), luciferase or betagalactosidase into cells capable of differentiating into pancreatic β cells.

### 12. Method of isolating cells capable of differentiating into pancreatic β cells using an antibody

As the method of obtaining cells expressing the desired surface antigen from various tissues taken out of a living organism, there may be mentioned the method using a flow cytometer having a sorting function, and the method using magnetic beads.

As the flow cytometer-based sorting system, there may be mentioned, among others, the waterdrop charging system and cell capture system [Furo Saitometa Jiyu-Jizai (Free and Easy Uses of Flow Cytometers), pp. 14-23, published 1999 by Shujunsha]. Both methods can quantify the antigen expression by converting the intensity of the fluorescence emitted from the antibody bound to a molecule expressed on the cell surface to an electric signal. Further, by using a combination of fluorescence species, it is also possible to utilize a plurality of surface antigens for separation. As the fluorescence species, there may be mentioned FITC (fluorescein isothiocyanate), PE (phycoerythrin), APC (Allo-phycocyanin), TR (Texas Red), Cy3, CyChrome, Red 613, Red 670, PerCP, TRI-Color, QuantumRed, etc. [Furo Saitometa Jiyu-Jizai (Free and Easy Uses of Flow Cytometers), pp. 3-13, published 1999 by Shujunsha].

As the method of staining, there may be mentioned the method comprising separating cells from various tissues taken out of the living body, specifically bone marrow or cord blood, by centrifugation, for instance, and directly staining them with an antibody, and the method comprising staining with an antibody after once cultivating and proliferating the cells in an appropriate medium.

For the cell staining, the target cell sample is first mixed with a surface antigen-recognizing primary antibody, and the mixture is incubated on ice for 30 minutes to 1 hour. When the primary antibody is a fluorescence-labeled one, the cells are washed and then separation carried out using a flow cytometer. When the primary antibody is a non-fluorescence-labeled one, the cells after reaction with the primary antibody are washed and mixed with a fluorescence-labeled secondary antibody having binding activity to the primary antibody, and the mixture is again incubated on ice for 30 minutes to 1 hour. After washing, the cells stained with the primary and secondary antibodies are separated using a flow cytometer.

The method using magnetic beads can separate a large amount of cells expressing the surface antigen in question. The separation purity is inferior as compared with the above-mentioned method using a flow cytometer but a sufficiently high level of cell purity can be secured by repeating the purification.

After reaction of cells with a primary antibody, that portion of the antibody remaining unreacted with cells is removed, and the cells are reacted with a magnetic bead-bound secondary antibody specifically binding to the primary antibody. The remaining secondary antibody is removed by washing and the cells can then be separated on a stand equipped with a magnet. The materials and apparatus necessary for these operations can be obtained from DYNAL.

The method using magnetic beads can be utilized also in removing unnecessary cells from the cell sample. For more efficient removal of unnecessary cells, the StemSep method available from Stem Cell Technologies Inc. (Vancouver, Canada) can be used.

As the antibodies to be used in the above methods, there may be mentioned the antibodies obtained as described above under 8, or antibodies recognizing CD34, CD117, CD14, CD45, CD90, Sca-1, Ly6c and Ly6g, which are surface antigens of hematopoietic system cells, antibodies recognizing Flk-1, CD31, CD105 and CD144, which are surface antigens of vascular endothelial cells, antibodies recognizing CD140, which is a surface antigen of mesenchymal cells, antibodies recognizing CD49b, CD49d, CD29 and CD41, which are surface antigens of integrin, and antibodies recognizing CD54, CD102, CD106 and CD44, which are matrix receptors. By using these antibodies in combination, it is possible to obtain the desired cells with higher purity.

More specifically, CD34-negative, CD117-positive, CD144-negative and CD140-positive cells, for instance, can be obtained by removing CD34-positive cells and CD144-positive cells from human bone marrow-derived mesenchymal cells by the above-mentioned immune magnetic bead method or the like and then collecting a CD117-positive and CD140-positive cell fraction.

### 13. Isolation of cells precursory of pancreatic β cells using a pancreatic β cell-specific gene promoter/reporter vector

For efficiently separating pancreatic β cells or cells precursory of pancreatic β cells as derived from cells capable of differentiating into pancreatic β cells, the green fluorescent protein (GFP) of Aequorea victoria can be used as a reporter gene indicator for gene transfer.

Specifically, a vector containing the GFP gene inserted at a site downstream of the promoter of a gene specifically expressed in pancreatic β cells or of a gene specifically expressed in the cells capable of differentiating into pancreatic β cells as obtained as described above under 9 is constructed and introduced into cells capable of differentiating into pancreatic p cells. The cells with such reporter vector introduced therein are separated using a drug resistance or a like indicator and then induced to differentiate into pancreatic β cells. The differentiation-induced cells express GFP and emit fluorescence. The fluorescence-emitting pancreatic β cells and pancreatic β cell precursor cells can be separated with ease using a flow cytometer [Furo Saitometa Jiyu-Jizai (Free and easy uses of flow cytometers), pp. 44-52, published 1999 by Shujunsha].

Employable as the promoter of the gene specifically expressed in pancreatic β cells are insulin, pax, etc.

Usable as the vector are, among others, the above-mentioned plasmid vectors and adenovirus vectors for animal cells.

### 14. Isolation of cells capable of differentiating into pancreatic β cells using Hoechst 33342

Hoechst 33342 is a DNA-binding dye and can stain living cells. A majority of bone marrow-derived mesenchymal cells are actively dividing, so that they are stained very brightly. Immature cells are stained dark. This is supported by the description in the literature (Nakauchi, Hiromitsu, Tanpakushitsu, Kakusan, Koso (Protein, Nucleic Acid, Enzyme), Vol. 45, No. 13, 2056-2062, 2000) that as the immaturity of cells increases, the ability to exclude the dye by an ABC (ATP binding cassette) transporter increases. Therefore, the cells capable of differentiating into pancreatic β cells according to the invention can be isolated by staining the test cells with Hoechst 33342 and separating unstained cells (with Hoechst 33342 not incorporated therein).

More specifically, bone marrow-derived mesenchymal cells, for instance, are stained with Hoechst 33342 and then subjected to short wavelength and long wavelength double staining analysis using a FACS under UV laser beam irradiation and, in this way, cells stained dark with Hoechst 33342 can be separated from the bone marrow. Immature cells that will not take up Hoechst 33342 can be fractionated as a side population [cf. Goodell, M. A. et al., J. Exp. Med., 183, 1797-1806 (1996), http://www.bcm.tmc.edu/genetherapy/goodell/new_site/index2.htm 1].

As described above in detail, the present invention provides mesenchymal cells and pancreatic β cells derived therefrom, which are effective in the treatment of impaired glucose tolerance-due diseases such as diabetes resulting from disruption and degeneration of pancreatic β cells and in searching for therapeutic agents for such diseases. Further, the invention provides pancreatic β cell forming agents, such as those cytokines, transcription factors, physiologically active substances and adhesion molecules/extracellular matrices which are useful in differentiation of said cells into pancreatic β cells, as well as methods of utilization thereof.

### BRIEF DESCRIPTION OF THE DRAWINGS

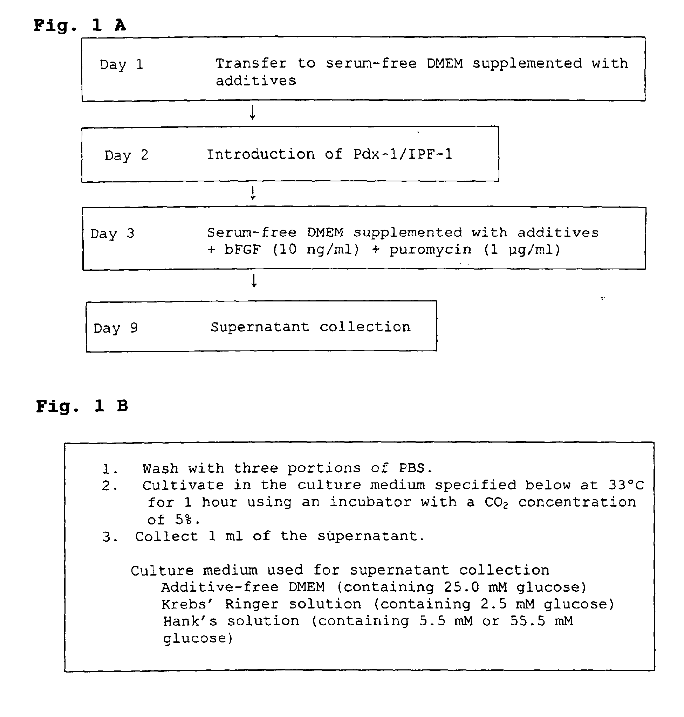

Fig. 1A is a flowchart showing a procedure for inducing the differentiation of mouse bone marrow-derived mesenchymal cells (KUSA/A1) into pancreatic β cells. KUSA/A1 cells were transfected with Pdx-1/IPF-1, and cultured in serum-free DMEM supplemented with 1 µg/ml of puromycin and 10 ng/ml of bFGF and, then, the supernatant was collected.
Fig. 1B is a representation of a procedure for supernatant collection and of the culture medium used for supernatant collection. After one week of cultivation, the culture medium in each dish was discarded, the cells in each dish were washed repeatedly three times with PBS and, after washing, the culture medium for supernatant collection, namely DMEM containing 25.0 mM glucose, Krebs' Ringer solution (KRS) containing 2.5 mM glucose, or Hank' s solution containing 5.5 mM or 55.5 mM glucose, was added, and cultivation was carried out at 33°C in an incubator with a CO₂ concentration of 5% for 1 hour. Then, 1 ml of the culture fluid was collected from each dish for insulin concentration determination.

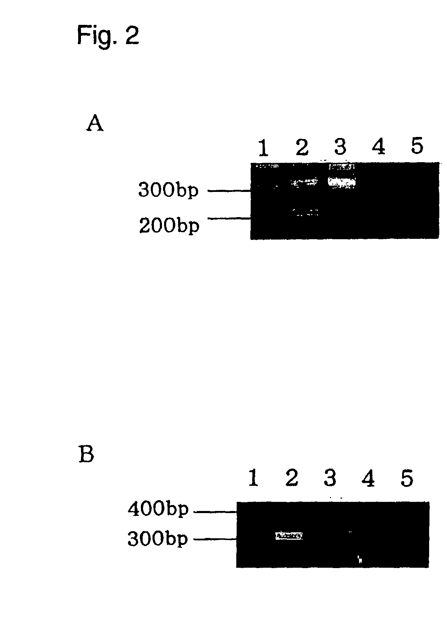

Fig. 2A and Fig. 2B are representations of the results of testing for the expression of the mouse preproinsulin gene I (197 bp) and mouse preproinsulin gene II (292 bp), respectively, in KUSA/A1 following transfection with Pdx-1/IPF-1 and the subsequent 1 week of cultivation in serum-free DMEM containing insulin, transferrin, progesterone, putrescine and selenium and supplemented with 10 ng/ml of bFGF. For said KUSA/A1, the expression of the mouse preproinsulin gene I (197 bp) and mouse preproinsulin gene II (292 bp) was observed (lane 2). For KUSA/A1 cultured in DMEM supplemented with 10% FBS (lane 3), a negative control with sterilized distilled water added in lieu of the template DNA (lane 4) and a negative control with sterilized distilled water added in lieu of the outer PCR product (lane 5), the expression of the mouse preproinsulin gene I or mouse preproinsulin gene II was not detected. Lane 1 shows the markers (100 bp ladder).

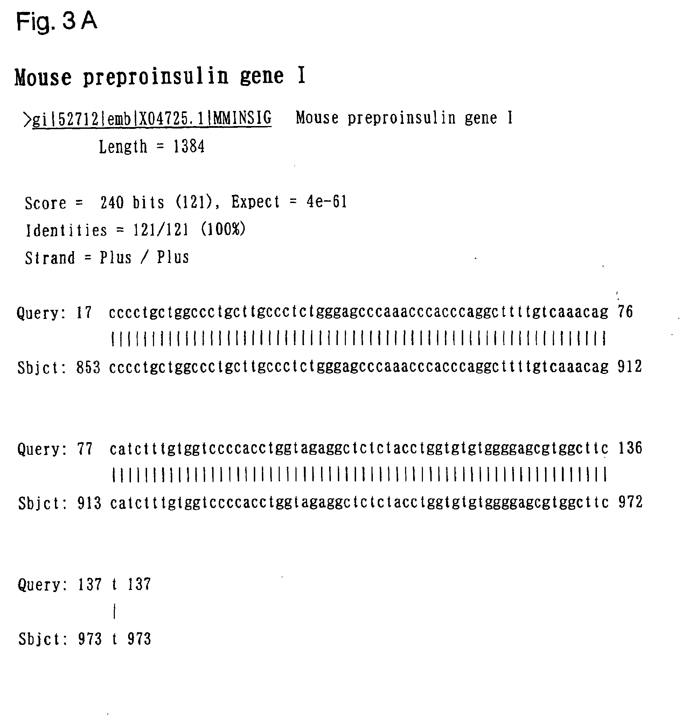

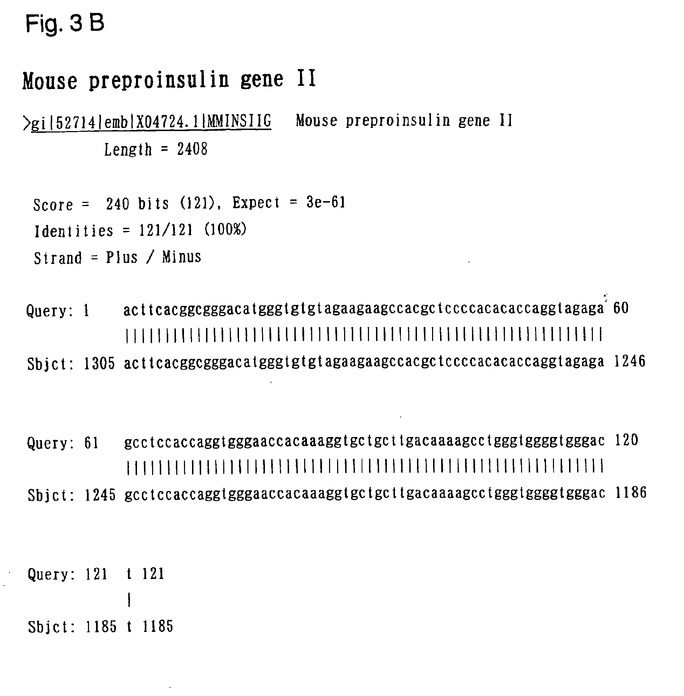

Fig. 3A and Fig. 3B show the results of base sequence determination of the PCR products extracted from the bands observed as a result of the PCR. The base sequences determined were searched for through NCBI nucleotide-nucleotide BLAST and, as a result, they were found to be 100% identical with the base sequences of mouse preproinsulin gene I and mouse preproinsulin gene II, respectively.

### BEST MODES FOR CARRYING OUT THE INVENTION

The following examples are given for illustrating the present invention in more detail. They are, however, by no means limitative of the scope of the present invention.

### Example 1: Preparation of bone marrow-derived mesenchymal cells capable of differentiating into pancreatic β cells frommouse bone marrow and cultivation thereof

Ten 5-week-old C3H/He mice were anesthetized with ether and then sacrificed by cervical dislocation. The mice were placed in hemilateral position and disinfected spraying with a sufficient amount of 70% ethanol.

Then, the skin around the femur was extensively cut, and the quadriceps muscle of thigh was excised all over the femur. The knee joint portion was cut with scissors to remove the joint, and, further, the muscle on the back of the femur was excised. The hip joint portion was cut with scissors, the joint was removed, and the femur was taken out. The muscle adhering to the femur was removed with scissors, and the whole femur was exposed. Both ends of the femur were cut off with scissors and, then, a 2.5-ml syringe equipped with a TERUMO 23G needle was filled with about 1.5 ml of IMDM supplemented with 20% of FCS, and the tip of the needle was inserted into the section, on the knee joint side, of the femur. The culture medium in the syringe was injected through the bone marrow into a test tube to thereby push out bone marrow-derived mesenchymal cells into the tube.

The cells obtained were cultivated in IMDM containing 20% FCS, 100 mg/ml penicillin, 250 ng/ml streptomycin and 85 mg/ml amphotericin at 33°C in an incubator with a CO₂ concentration of 5%. After repetitions of cell passage, all cells were equally mesenchymal cells as a result of disappearance of hematopoietic system cells.

The cultivation was carried out under the above conditions for about 4 months and immortalized cells were thereby selected and, then, 192 independent single-cell derived cell lines were obtained by dilution. One of bone marrow-derived mesenchymal cell lines capable of differentiating into pancreatic β cells among these cell lines was named "KUSA/A1". Hereafter, unless otherwise specified, KUSA/Al bone marrow-derived mesenchymal cells were cultivated in DMEM containing 10% FCS, 100 mg/ml penicillin and 250 ng/ml streptomycin at 33°C in an incubator with a CO₂ concentration of 5%.

### Example 2: Surface antigen analysis of KUSA/A1 cells

For isolating and purifying cells capable of efficiently differentiating into pancreatic β cells from the bone marrow, KUSA/A1 cells were analyzed for surface antigens.

For the analysis, 20 surface antigens were used, namely CD105, Flk-1, CD31 and CD144, which are known to be surface antigens of vascular epithelial cells, CD34, CD117 (c-kit), CD14, CD45, CD90, Ly6A/E (Sca-1), Ly6c and Ly6g, which are known to be surface antigens of hematopoietic system cells, CD140 known to be a mesenchymal cell surface antigen, integrins CD49b, CD49d and CD29, and matrix receptors CD54, CD102, CD106 and CD44.

First, KUSA/A1 cells were distributed into the wells of a 96-well U-shaped plate (1 x 10⁴ cells/well). An anti-mouse CD105 antibody (product of Pharmingen) labeled with biotin by the conventional method [Koso Kotaiho (Immunoenzyme Technique), published by Gakusai Kikaku (1985)] was added to a buffer for FACS (1% BSA-PBS, 0.02% EDTA, 0.05% NaN₃, pH 7.4) and added to each well, and the reaction was allowed to proceed on ice for 30 minutes. Purified rat IgG2a,κ antibody (product of Pharmingen) was used as a control antibody. After washing with two portions of the buffer, 20 µl of streptavidin-PE (product of Japan Becton Dickinson) was added. The reaction was allowed to proceed on ice for 30 minutes while light was shielded and, after washing with three portions of the buffer, the cells were suspended in a final amount of 500 µl, the fluorescence intensity was measured using a flow cytometer, and the occurrence or nonoccurrence of antibody expression was judged according to whether the fluorescence intensity increased as a result of addition of the antibody or not. As a result, it was established that KUSA/A1 cells are negative for CD105.

As for the expression of the Flk-1 antigen, the antibody reaction was carried out in the same manner as mentioned above using a biotinylated anti-mouse Flk-1 antibody (product of Pharmingen; PM-28181D), and measurements were made using a flow cytometer. As a result, it was established that KUSA/A1 cells are negative for Flk-1.

As for the expression of the CD31 antigen, the antibody reaction was carried out using a FITC-labeled anti-mouse CD31 antibody (product of Pharmingen; PM-01954 D) , and measurements were made using a flow cytometer. As a result, it was established that KUSA/A1 cells are negative for CD31.

As for the expression of the CD144 antigen, the antibody reaction was carried out using a biotinylated anti-mouse CD144 antibody (product of Pharmingen; PM-28091D), and measurements were made using a flow cytometer. As a result, it was established that KUSA/A1 cells are CD144-negative cells.

As for the expression of the CD34 antigen, the antibody reaction was carried out using a FITC-labeled anti-mouse CD34 antibody (product of Pharmingen; PM-09434D) , andmeasurementswere made using a flow cytometer. As a result, it was established that KUSA/A1 cells are negative for CD34.

As for the expression of the CD117 (c-kit) antigen, the antibody reaction was carried out using a FITC-labeled anti-mouse CD117 antibody (product of Pharmingen; PM-01904D), and measurements were made using a flow cytometer. As a result, it was established that KUSA/A1 cells are negative for CD117.

As for the expression of the CD14 antigen, the antibody reaction was carried out using a FITC-labeled anti-mouse CD14 antibody (product of Pharmingen; PM-09474), and measurements were made using a flow cytometer. As a result, it was established that KUSA/A1 cells are positive for CD14 and that BMSC cells are negative for CD14.

As for the expression of the CD45 antigen, the antibody reaction was carried out using a FITC-labeled anti-mouse CD45 antibody (product of Pharmingen; PM-01114), and measurements were made using a flow cytometer. As a result, it was established that KUSA/A1 cells are negative for CD45.

As for the expression of the CD90 antigen, the antibody reaction was carried out using a FITC-labeled anti-mouse CD90 antibody (product of Pharmingen; PM-22214), and measurements were made using a flow cytometer. As a result, it was established that KUSA/A1 cells are negative for CD90.

As for the expression of the Ly6A/E (Sca-1) antigen, the antibody reaction was carried out using a FITC-labeled anti-mouse Ly6A/E (Sca-1) antibody (product of Pharmingen; PM-01164A), and measurements were made using a flow cytometer. As a result, it was established that KUSA/A1 cells are positive for Ly6A/E (Sca-1).

As for the expression of the Ly6c antigen, the antibody reaction was carried out using a FITC-labeled anti-mouse Ly6c antibody (product of Pharmingen; PM-01152), and measurements were made using a flow cytometer. As a result, it was established that KUSA/A1 cells are positive for Ly6c.

As for the expression of the Ly6g antigen, the antibody reaction was carried out using a FITC-labeled anti-mouse Ly6g antibody (product of Pharmingen; PM-01214), and measurements were made using a flow cytometer. As a result, it was established that KUSA/A1 cells are negative for Ly6g.

As for the expression of the CD140 antigen, the antibody reaction was carried out using a biotinylated anti-mouse CD140 antibody (product of Pharmingen; PM-28011A), and measurements were made using a flow cytometer. As a result, it was established that KUSA/A1 cells are positive for CD140.

As for the expression of the CD49b antigen, the antibody reaction was carried out using a FITC-labeled anti-mouse CD49b antibody (product of Pharmingen; PM-09794), and measurements were made using a flow cytometer. As a result, it was established that KUSA/A1 cells are positive for CD49b.

As for the expression of the CD49d antigen, the antibody reaction was carried out using a FITC-labeled anti-mouse CD49d antibody (product of Pharmingen; PM-01274), and measurements were made using a flow cytometer. As a result, it was established that KUSA/A1 cells are negative for CD49d.

### Example 3: Induction of differentiation of bone marrow-derived mesenchymal cells into pancreatic β cells - (1)

The KUSA/A1 cells obtained in Example 1 were treated with trypsin and transferred to a 6-well dish (product of Falcon) so that the cell density might become as low as possible.

The cells were cultured in serum-free DMEM containing insulin, transferrin, progesterone, putrescine and selenium or in DMEM containing 10% FBS at 33°C for 12 days using an incubator with a CO₂ concentration of 5%. bFGF (recombinant human basic fibroblast growth factor; product of R&D) was added to each of the serum-free or serum-containing wells to a final concentration of 10 ng/ml. Hereafter, unless otherwise specified, cell culture was carried out under the same conditions while medium exchange was conducted at 2-day intervals.

After 12 days of cultivation, the medium in each well was discarded, the cells in each well were washed repeatedly with three portions of PBS. After washing, 2 ml of PBS was added to each well afresh, and cultivation was performed at 33°C for 2 hours using an incubator with a CO₂ concentration of 5%.

After 2 hours of cultivation, the PBS was discarded, 2 ml of DMEM containing 25.0 mM glucose was added afresh, and cultivation was performed at 33°C for further 2 hours using an incubator with a CO₂ concentration of 5%.

At 2 hours after addition of glucose, the cultivation was finished and, then, about 2 ml of the supernatant in each well was collected into a tube (collection tube) using a pipette.

Each of the thus-obtained supernatant samples was assayed for insulin concentration by the ELISA method (insulin assaying kit, product of Morinaga).

As a result, 223 pg/ml of insulin was detected in the supernatant obtained by cell culture in serum-free DMEM containing bFGF, insulin, transferrin, progesterone, putrescine and selenium. On the other hand, insulin was not detected in the supernatant obtained by cell culture in DMEM containing bFGF and 10% FBS (below assay sensitivity limit, namely below 39 pg/mL).

Further, for the supernatant obtained by cell culture in serum-free, bFGF-free DMEM containing insulin, transferrin, progesterone, putrescine and selenium, 51.9 pg/ml of insulin was detected. On the contrary, insulin was not detected in the supernatant obtained by cell culture in bFGF-free, 10% FBS-containing DMEM (below assay sensitivity limit).

In control runs, the PBS used for washing wells and the DMEM containing 25. 0 mM glucose as added to each well after washing were assayed for insulin concentration by the same method; the insulin concentration in them were below the assay sensitivity limit.

### Example 4. Induction of differentiation of bone marrow-derived mesenchymal cells into pancreatic β cells - (2)

A plasmid vector, pCAGIPuro pdx, constructed by inserting the pancreatic duodenal homeobox 1 gene (Pdx-1/IPF-1) specified in the sequence listing under SEQ ID NO:1 into a plasmid vector, pCAGIPuro, with the puromycin resistance gene inserted therein was allowed to be taken up by competent Escherichia coli cells for transformation thereof, and plasmid preparation was performed using QIAGEN Plasmid Maxi Kits (product of Quiagen).

The KUSA/A1 cells obtained in Example 1 were transfected with the transcription factor Pdx-1/IPF-1 by the lipofection method using FuGENE transfection reagent (product of Roche) and cultured in serum-free DMEM containing insulin, transferrin, progesterone, putrescine and selenium and supplemented with 1 µg/ml puromycin and 10 ng/ml bFGF at 33°C for 1 week using an incubator with a CO₂ concentration of 5%. The cell culture was performed while medium exchange was performed at 2-day intervals. The protocol for inducing the differentiation of KUSA/A1 into pancreatic β cells is shown in Fig. 1A.

After 1 week of cultivation, the culture medium in each dish was discarded, the cells in each dish were washed repeatedly with three portions of PBS and, after washing, 2 ml or 4 ml of a culture medium for supernatant collection, namely DMEM containing 25.0 mM glucose, Krebs' Ringer solution (KRS) containing 2.5 mM glucose or Hank's solution containing 5.5 mM or 55.5 mM glucose, was added to a 6 cm or 10 cm dish, respectively, and cultivation was performed at 33°C for 1 hour using an incubator with a CO₂ concentration of 5%. After 1 hour of cultivation, 1 ml of the culture fluid was collected from each dish and assayed for insulin concentration. The procedure for supernatant collection is shown in Fig. 1B.

As a result, the insulin content in the serum-free DMEM containing insulin, transferrin, progesterone, putrescine and selenium was 151-268 pg/ml. The DMEM, Krebs' Ringer solution and Hank's solution used for supernatant collection each showed an insulin content below the assay sensitivity limit (below 39 pg/ml). On the contrary, in each of the culture supernatants obtained by cell culture in serum-free DMEM containing insulin, transferrin, progesterone, putrescine and selenium and supplemented with bFGF and employed for supernatant collection, insulin was detected and the highest level thereof was 2200 pg/ml. The results are summarized in Table 1.

**Table 1**

| Insulin concentrations in cell culture supernatants after 1 week or 2 weeks of cultivation of KUSA/A1 in serum-free DMEM according to the protocol | | |
|---|---|---|
| No. | Medium | Insulin (pg/ml) |
| Exp.1 | Hank's Solution, 50mM glucose | 771 |
| | | 625 |
| | | 496 |
| | | 383 |
| Exp.2 | DMEM, 54.8mM glucose | 119 |
| | | 141 |
| | | 208 |
| Exp.3 | Hank's Solution, 50mM glucose | 2200 |
| | | 1500 |
| | | 1190 |
| Exp.4 | KRS, 2.6mM glucose, 10% FBS | 798 |
| | | 764 |
| Exp.5 | DMEM, 54.8mM glucose | 1440 |
| | | 402 |
| Exp.6 | Hank's solution, 5.6mM glucose | 389 |
| | | 349 |

RNA was obtained, using ISOGEN (Nippon Gene), from KUSA/A1 transfected with Pdx-1/IPF-1 and cultivated in serum-free DMEM containing insulin, transferrin, progesterone, putrescine and selenium and supplemented with 1 µg/ml puromycin and 10 ng/ml bFGF at 33°C for 1 week using an incubator with a CO₂ concentration of 5%. Then, a first strand cDNA was prepared from the RNA using a first-strand cDNA synthesis kit (Amersham Pharmacia Biotech). Then, nested RT-PCR was carried out for the mouse preproinsulin gene I and mouse preproinsulin gene II using rTaq DNA polymerase (Toyobo) with the first strand cDNA constructed as a substrate. Synthetic DNAs having the respective base sequences shown in Table 2 were used for the amplification of the mouse preproinsulin gene I and mouse preproinsulin gene II. The RT-PCR conditions and the primers used are shown in Table 2.

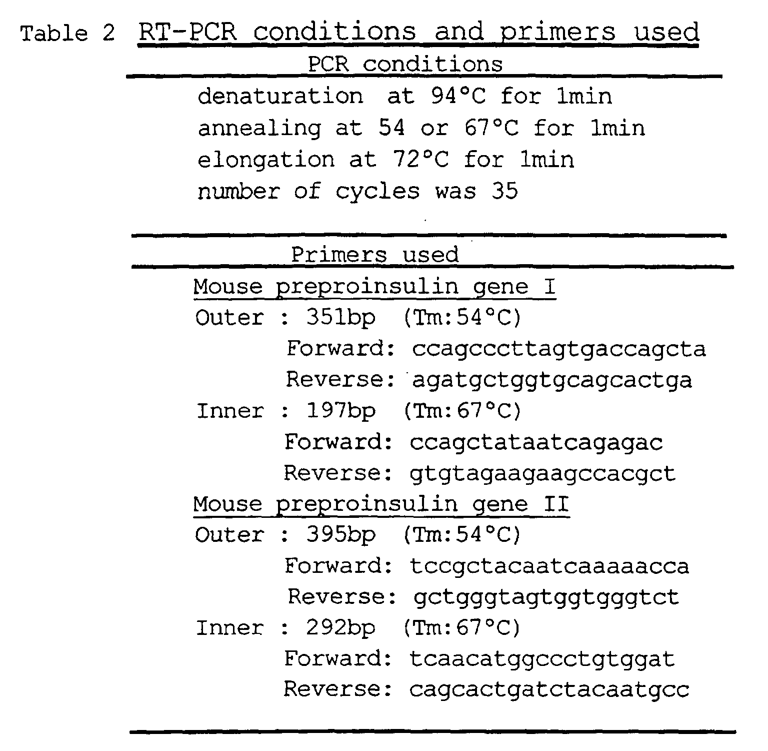

As a result, the expression of the mouse preproinsulin gene I (197 bp) and mouse preproinsulin gene II (292 bp) was observed for KUSA/A1 cells transfected with Pdx-1/IPF-1 and cultured in serum-free DMEM containing insulin, transferrin, progesterone, putrescine and selenium and supplemented with 10 ng/ml bFGF for 1 week (lane 2). The expression of the mouse preproinsulin gene I or mouse preproinsulin gene II was not observed for KUSA/A1 cells cultured in DMEM supplemented with 10% FBS (lane 3), a negative control with sterilized distilled water added in lieu of the template DNA (lane 4) and a negative control with sterilized distilled water added in lieu of the outer PCR product (lane 5). For lane 1 markers (100 bp ladder) were used. The results are shown in Fig. 2A and Fig. 2B.

The PCR products were extracted from the bands observed as a result of PCR and their base sequences were determined. The base sequences determined were searched for through NCBI nucleotide-nucleotide BLAST and, as a result, they were found to be 100% identical with a part of the base sequence of the mouse preproinsulin gene I and a part of the base sequence of the mouse preproinsulin gene II, respectively. The results are shown in Fig. 3A and Fig. 3B.

### INDUSTRIAL APPLICABILITY

The present invention provides mesenchymal cells and pancreatic β cells derived therefrom, which are effective in the treatment of impaired glucose tolerance-due diseases such as diabetes resulting from disruption and degeneration of pancreatic β cells and in searching for therapeutic agents for such diseases. Further, the invention provides pancreatic p cell forming agents, such as those cytokines, transcription factors, physiologically active substances and adhesion molecules/extracellular matrices which are useful in differentiation of said cells into pancreatic β cells, as well as methods of utilization thereof.

## Claims

1. A method of forming pancreatic β cells from mesenchymal cells which comprises subjecting mammal-derived mesenchymal cells to at least one step selected from the following steps (a) to (e) :
a) the step of proliferating mesenchymal cells obtained from a mammalian sample and capable of differentiating into pancreatic β cells;
b) the step of selecting and isolating those mesenchymal cells which are capable of differentiating into pancreatic β cells from among mesenchymal cells obtained from a mammalian sample or from among the mesenchymal cells obtained in step a) using an antibody or antibodies capable of binding to a cell membrane antigen;
c) the step of cultivating the mesenchymal cells capable of differentiating into pancreatic β cells as obtained in step a) or b) or cells including such mesenchymal cells in an adhesion molecule/extracellular matrix-coated reaction vessel which enables the cells to contact with the adhesion molecules/extracellular matrix;
d) the step of cultivating the mesenchymal cells capable of differentiating into pancreatic β cells as obtained in step a), b) or c) or cells including such mesenchymal cells in contact with a pancreatic β cell-forming agent; and
e) the step of selecting and separating the pancreatic β cells obtained in the step c) or d) using a gene specifically expressed in pancreatic β cells as a selective marker.

2. A method of forming pancreatic β cells from mesenchymal cells as defined in Claim 1, wherein the mesenchymal cells are obtained from bone marrow, muscle, pancreas, liver, small intestine, large intestine, kidney, subcutaneous tissue, endometrium, blood, cord blood or placenta.

3. A method of forming pancreatic β cells from mesenchymal cells as defined in Claim 1 or 2, wherein the selection of mesenchymal cells in step b) is carried out using a CD140-positive antibody.

4. A method of forming pancreatic β cells from mesenchymal cells as defined in any of Claims 1 to 3, wherein, in step e), the gene specifically expressed in pancreatic β cells is the insulin gene.

5. A method of forming pancreatic β cells from mesenchymal cells as defined in any of Claims 1 to 4, wherein, in step d), the pancreatic β cell-forming agent comprises at least one member selected from the group consisting of cytokines, physiologically active substances, transcription factors and adhesion molecules/extracellular matrices.

6. A method of forming pancreatic β cells from mesenchymal cells as defined in Claim 5, wherein the cytokine selected comprises at least one member selected from the group consisting of hepatocyte growth factor (HGF), fibroblast growth factor (bFGF)/FGF-2, insulin, transferrin, heparin-binding EGF, gastrin, TGF-β, insulin-like growth factor (IGF-1), parathyroid hormone-related proteins (PTHrP), growth hormone, prolactin, placental lactogen, glucagon-like peptide-1, exendin-4 and KGF (keratinocyte growth factor).

7. A method of forming pancreatic β cells from mesenchymal cells as defined in Claim 5, wherein the physiologically active substance selected comprises at least one member selected from the group consisting of nicotinamide, betacellulin, activin A, progesterone, putrescine and selenium.

8. A method of forming pancreatic β cells from mesenchymal cells as defined in Claim 5, wherein the transcription factor selected comprises at least one member selected from the group consisting of PTF1a/PTF-P48, Isl-1, Pdx-1/IPF-1, Beta2/neuroD, ngn3, PAX-6, PAX-4, Hlxb-9, Nkx2.2, Nkx6.1, HNF1α, HNF1β and HNF4α.

9. A method of forming pancreatic β cells from mesenchymal cells as defined in Claim 5, wherein the adhesion molecule/extracellular matrix selected comprises at least one member selected from the group consisting of gelatin, laminin, collagen, agarose, fibronectin and ornithine.

10. A pancreatic β cell-forming agent for use in the method as defined in any of Claims 1 to 9 which comprises, as an active ingredient, at least one member selected from the group consisting of cytokines, physiologically active substances, transcription factors and adhesion molecules/extracellular matrices.

11. A pancreatic β cell-forming agent as defined in Claim 10, wherein the cytokine selected comprises at least one member selected from the group consisting of hepatocyte growth factor (HGF), fibroblast growth factor (bFGF)/FGF-2, insulin, transferrin, heparin-binding EGF, gastrin, TGF-β, insulin-like growth factor (IGF-1), parathyroid hormone-related proteins (PTHrP), growth hormone, prolactin, placental lactogen, glucagon-like peptide-1, exendin-4 and KGF.

12. A pancreatic β cell-forming agent as defined in Claim 10, wherein the physiologically active substance selected comprises at least one member selected from the group consisting of nicotinamide, betacellulin, activin A, progesterone, putrescine and selenium.

13. A pancreatic β cell-forming agent as defined in Claim 10, wherein the transcription factor selected comprises at least one member selected from the group consisting of PTF1a/PTF-P48, Isl-1, Pdx-1/IPF-1, Beta2/neuroD, ngn3, PAX-6, PAX-4, Hlxb-9, Nkx2.2, Nkx6.1, HNF1α, HNF1β and HNF4α.

14. A pancreatic β cell-forming agent as defined in Claim 10, wherein the adhesion molecule/extracellular matrix selected comprises at least one member selected from the group consisting of gelatin, laminin, collagen, agarose, fibronectin and ornithine.

15. A method of screening candidate substances promoting the formation of pancreatic β cells from mesenchymal cells which comprises causing the formation of pancreatic β cells from mesenchymal cells according to the method as defined in Claim 1 in the presence of each candidate substance and selecting a candidate substance showing a pancreatic β cell formation-promoting effect in comparison with pancreatic β cells formed in the absence of the candidate substance.

16. A method of screening candidate substances promoting the formation of pancreatic β cells from mesenchymal cells as defined in Claim 15, wherein the mesenchymal cells are obtained from bone marrow, muscle, pancreas, liver, small intestine, large intestine, kidney, subcutaneous tissue, endometrium, blood, cord blood or placenta.

17. A method of screening candidate substances promoting the formation of pancreatic β cells from mesenchymal cells as defined in Claim 15 or 16, wherein the selection of mesenchymal cells in step b) is carried out using a CD140-positive antibody.

18. A method of screening candidate substances promoting the formation of pancreatic β cells from mesenchymal cells as defined in any of Claims 15 to 17, wherein, in step e) , the gene specifically expressed in pancreatic β cells is the insulin gene.

19. A method of screening candidate substances promoting the formation of pancreatic β cells from mesenchymal cells as defined in any of Claims 15 to 18, wherein, in step d), the pancreatic β cell-forming agent comprises at least one member selected from the group consisting of cytokines, physiologically active substances, transcription factors and adhesion molecules/extracellular matrices.

20. A method of screening candidate substances promoting the formation of pancreatic β cells from mesenchymal cells as defined in Claim 19, wherein the cytokine selected comprises at least one member selected from the group consisting of hepatocyte growth factor (HGF), fibroblast growth factor (bFGF)/FGF-2, insulin, transferrin, heparin-binding EGF, gastrin, TGF-β, insulin-like growth factor (IGF-1), parathyroid hormone-related proteins (PTHrP), growth hormone, prolactin, placental lactogen, glucagon-like peptide-1, exendin-4 and KGF.

21. A method of screening candidate substances promoting the formation of pancreatic β cells from mesenchymal cells as defined in Claim 19, wherein the physiologically active substance selected comprises at least one member selected from the group consisting of nicotinamide, betacellulin, activin A, progesterone, putrescine and selenium.

22. A method of screening candidate substances promoting the formation of pancreatic β cells from mesenchymal cells as defined in Claim 19, wherein the transcription factor selected comprises at least one member selected from the group consisting of PTF1a/PTF-P48, Isl-1, Pdx-1/IPF-1, Beta2/neuroD, ngn3, PAX-6, PAX-4, Hlxb-9, Nkx2.2, Nkx6.1, HNF1α, HNF1β and HNF4α.

23. A method of screening candidate compounds promoting the formation of pancreatic β cells from mesenchymal cells as defined in Claim 19, wherein the adhesion molecule/extracellular matrix selected comprises at least one member selected from the group consisting of gelatin, laminin, collagen, agarose, fibronectin and ornithine.

24. A method of screening candidate substances promoting the formation of pancreatic β cells from mesenchymal cells as defined in any of Claims 15 to 23, wherein the candidate substance is a cultivation-derived composition.

25. A substance capable of promoting the formation of pancreatic β cells from mesenchymal cells as obtained by the method of screening candidate compounds promoting the formation of pancreatic β cells from mesenchymal cells as defined in any of Claims 15 to 24.

26. A method for treating an impaired glucose tolerance-due disease of a patient which comprises administering an effective amount of the pancreatic β cells obtained by the method as defined in any of Claims 1 to 9 to the patient.

27. A therapeutic agent for an impaired glucose tolerance-due disease which comprises, as an active ingredient, the pancreatic β cells obtainable by the method as defined in any of Claims 1 to 9.

28. A method of causing differentiation into insulin-secreting cells which comprises scattering cells capable of differentiating into insulin-secreting cells on the layer of mesenchymal cells as obtained by monolayer culture on a culture dish and carrying out the cocultivation thereof.

29. A method of causing differentiation into insulin-secreting cells by cocultivation as defined in Claim 28, wherein the cells capable of differentiating into insulin-secreting cell comprise at least one member selected from the group consisting of embryonic stem cells, pancreatic stem cells, small intestinal epithelial stem cells, liver-derived stem cells and amniotic cells.
